# EUROPEAN PATENT SPECIFICATION

(11) **EP 1 687 435 B1**
(45) Date of publication and mention of the grant of the patent: **28.09.2011**
(21) Application number: 04818796.7
(22) Date of filing: 17.11.2004
(51) Int. Cl.: C12N 15/113, C12Q 1/68

(54) **INSECT RESISTANCE USING INHIBITION OF GENE EXPRESSION**
INSEKTENRESISTENZ DURCH INHIBIERUNG VON GENEXPRESSION
RESISTANCE AUX INSECTES PAR INHIBITION DE L'EXPRESSION GENIQUE

(30) Priority: 17.11.2003 US 520306 P
(43) Date of publication of application: 09.08.2006
(73) Proprietor: Commonwealth Scientific and Industrial Research Organisation, Campbell, ACT 2601 (AU); Bayer BioScience N.V., 9052 Gent (BE)
(72) Inventor: WATERHOUSE, Peter Michael, O'Connor, Australian Capital Territory 2602 (AU); WHYARD, Steven, Winnipeg, Manitoba R3T2N2 (CA); VAN RIE, Jeroen, B-9900 Eeklo (BE)
(74) Representative: Meulemans, Wouter L. J.
(86) International application number: PCT/EP2004/013049
(87) International publication number: WO 2005/049841

(56) References cited:
- WO-A-00/01846
- WO-A-01/34815
- WO-A-01/37654
- WO-A-03/004644
- WO-A-03/076619
- WO-A2-2007/080126
- CA-A1- 2 094 658
- DATABASE EMBL M.persicae ecdysone receptor 11 October 1999 (1999-10-11), XP002317945 retrieved from EBI Database accession no. AAX90669 & WO 99/36520 A (COMMONWEALTH SCIENTIFIC AND INDUSTRIAL RESEARCH ORGANISATION; HILL, RO) 22 July 1999 (1999-07-22)
- TIMMONS L ET AL: "Specific interference by ingested dsRNA" NATURE, MACMILLAN JOURNALS LTD. LONDON, GB, vol. 395, no. 6705, 29 October 1998 (1998-10-29), page 854, XP002103601 ISSN: 0028-0836
- FIRE A: "RNA-triggered gene silencing" TRENDS IN GENETICS, ELSEVIER SCIENCE PUBLISHERS B.V. AMSTERDAM, NL, vol. 15, no. 9, 1 September 1999 (1999-09-01), pages 358-363, XP004176656 ISSN: 0168-9525
- MONTGOMERY M K ET AL: "Double-stranded RNA as a mediator in sequence-specific genetic silencing and co-suppression" TRENDS IN GENETICS, ELSEVIER SCIENCE PUBLISHERS B.V. AMSTERDAM, NL, vol. 14, no. 7, July 1998 (1998-07), pages 255-258, XP004124680 ISSN: 0168-9525
- MANOHARAN M: "Oligonucleotide conjugates as potential antisense drugs with improved uptake, biodistribution, targeted delivery, and mechanism of action" ANTISENSE & NUCLEIC ACID DRUG DEVELOPMENT, MARY ANN LIEBERT, INC., NEW YORK, US, vol. 12, 2002, pages 103-128, XP002294027 ISSN: 1087-2906
- GURA T: "A silence that speaks volumes.", NATURE 20 APR 2000 LNKD- PUBMED:10786767, vol. 404, no. 6780, 20 April 2000 (2000-04-20), pages 804-808, ISSN: 0028-0836
- RAJAGOPAL R ET AL: "Silencing of midgut aminopeptidase N of Spodoptera litura by double-stranded RNA establishes its role as Bacillus thuringiensis toxin receptor.", THE JOURNAL OF BIOLOGICAL CHEMISTRY 6 DEC 2002 LNKD- PUBMED:12377776, vol. 277, no. 49, 6 December 2002 (2002-12-06), pages 46849-46851, ISSN: 0021-9258
- FUTAKI ET AL: 'Stearylated Arginine-Rich peptides: A new class of transfection systems' BIOCONJUGATE CHEM vol. 12, 27 October 2001, pages 1005 - 1011

### SEQUENCE LISTING

<110> Commonwealth scientific and Industrial Research Organisation
   Bayer Bioscience NV
   Waterhouse, Peter
   Whyard, Steven
   Van Rie, Jeroen
<120> Insect resistance using inhibition of gene expression
<130> BCS03-2008 w01
<150> US 60/520,306
<151> 2003-11-17
<160> 12
<170> PatentIn version 3.0
<210> 1
<211> 27
<212> DNA
<213> artificial
<220>
<223> designed degenerate primer
<400> 1
   aaaacagaag aagaggtaaa aaygara 27
<210> 2
<211> 28
<212> DNA
<213> artificial
<220>
<223> designed degenerate primer
<220>
<221> misc_feature
<223> n at 20 is c, g, a or t
<400> 2
   ggtttctggc ttcgtctggn gtrtaytt 28
<210> 3
<211> 20
<212> DNA
<213> artificial
<220>
<223> designed degenerate primer
<400> 3
   tacaactcsa tcytgaccac 20
<210> 4
<211> 22
<212> DNA
<213> artificial
<220>
<223> designed degenerate primer
<400> 4
   tccatrccyt cwccbacrta cc 22
<210> 5
<211> 279
<212> DNA
<213> Aphis gossypii
<400> 5
   aaaacagaag aagaggtaaa aatgagaatg aaacagaaaa gcgtgagttg gtgttcaaag 60
   aagatggaca agaatatgct caagttacca aaatgttggg aaatggacgt ctagaagcaa 120
   tgtgttttga tggtgtaaga cgactttgtc acattcgagg aaaacttagg aaaaaggtgt 180
   ggatcaatca agctgacata gtattgatag gcttacgtga atatcaagat acaaaagccg 240
   atgtaatttt gaaatacacc ccagacgaag ccagaaacc 279
<210> 6
<211> 279
<212> DNA
<213> Myzus persicae
<400> 6
   aaaacagaag aagaggtaaa aacgaaaatg aaaccgagaa gcgtgaattg gtgttcaaag 60
   aagatggcca agaatatgct caagttacca aaatgttggg aaatggacgt ctagaagcta 120
   tgtgctttga tggtgttaaa cgactttgcc acatacgagg aaaacttagg aaaaaggtat 180
   ggattaatca agctgatata gtattaatag gtttacgtga ataccaagac acaaaagccg 240
   atgtaatttt gaaatacaca ccagacgaag ccagaaacc 279
<210> 7
<211> 638
<212> DNA
<213> Aphis gossypii
<220>
<221> misc_feature
<223> n at 591, 592 and 637 is a, c, g or t
<400> 7
   tcatggctgg actacgaggc catctacgac atctgccgcc ggaacctgga catcgagcgg 60
   cccacgtaca cgaacctcaa ccggctgatc gggcagatcg tgtcgttccc atcacgggcg 120
   tcgctgcggt tcgacggcgc gctgaacgtc gacctgaccg agttccagac gaacctggtg 180
   ccgtacccgc gcattcactt cccgctggcc acgtacgcgc cggtcatatc ggccgagaag 240
   gcgtaccacg agcagctgtc cgtggccgaa atcaacccaa cgcgtgcttc gaaccggcca 300
   aaccagaatg ggtcaagatg cgacccgcgg cacggcaagt acatgggcct gctgcaatgc 360
   tgtaaccgcg gcgacgtcgt gcccaaggac atgaacgcgg ccatcgccac catcaagacc 420
   aagaggacca tcgtgtacgt cgactggtgc ccgaccgggt tcaaggtggg catctactac 480
   cagccgccga ccgtggtgcc gggggcgatc tggccaaggt gcagcgggcg gtgtgcatgt 540
   tgtccaacac gacggccatc tccgaggcgt gggcccggct cgaccacaag nntgacctga 600
   tgtacgctga cacgcgcgtc cgtccactgg tacgtang 638
<210> 8
<211> 628
<212> DNA
<213> Myzus persicae
<220>
<221> misc_feature
<223> n at 3, 113, 128, 137, 509, 615, 617, and 627 is a, c, g, or t
<400> 8
   tgnacacagt gactgtgcat tcatggtcga taatgaagcc atctatgaca tctgccgtcg 60
   taatctcgat attgaacgtg cccacttaca ctaacttgaa tcgtcttatt ggncagattg 120
   tgtcttcnat cacagcntct ctccgtttcg atggtgccct caatgttgac ttgactgaat 180
   tccagaccaa tttggtccca tacccccgta ttcatttccc attggtcact tatgcaccag 240
   tcatctccgc tgaaaaggct taccatgaac aattgtccgt atcagaaatc actaacgctt 300
   gttttgaacc agccaaccaa atggtgaaat gtgatccacg tcatggcaaa tacatggctt 360
   gttgcatgtt gtaccgtggt gatgttgtac ccaaagacgt caacgctgcc attgcttcca 420
   tcaagaccaa gagaacattc agtttgttga ctggtgtcca actggtttca aagttgggta 480
   tcaactacca acccccaacc gtggtaccng gtgtgacttg gtctaaagta caacgtgctg 540
   tctgcatgtt gtccaacact acagctattg ctgaagcttg ggtctaggtt tggtaccaca 600
   agttcgtaac ttgcnantac gtccacna 628
<210> 9
<211> 30
<212> DNA
<213> artificial
<220>
<223> designed primer sequence
<400> 9
   cccaagcttt gcctggtgtg tggcgaccgg 30
<210> 10
<211> 30
<212> DNA
<213> artificial
<220>
<223> designed primer sequence
<400> 10
   cccaagctta tcctggaaat agacaagtcg 30
<210> 11
<211> 408
<212> DNA
<213> Myzus persicae
<400> 11
   tcgtccggtt accattacaa cgctctcaca tgcgaaggat gcaaggggtt cttccggagg 60
   agcatcacca agaacgccgt gtaccagtgc aagtacggca acaattgcga aatcgacatg 120
   tacatgaggc ggaagtgcca ggagtgccgg ctgaaaaaat gcctgaccgt cggcatgagg 180
   cctgaatgtg ttgtacctga agttcaatgc gcagtaaaaa gaaaggagaa aaaagctcaa 240
   cgagaaaaag ataaaccaaa ttctactaca gacatttctc ctgaaataat aaaaatagaa 300
   cctacagaga tgaagattga atgtggtgaa ccaatgataa tgggcacacc tatgccgact 360
   gtaccttacg tgaaaccttt gagttctgaa caaaaagaac tgatccac 408
<210> 12
<211> 1173
<212> DNA
<213> Myzus persicae
<220>
<221> misc_feature
<223> n at 704 is c, g, a, or t
<400> 12
   tgccagcgca tttgcgacca gtgggacaga ttaggtagct tgacacagaa acggagaact     60
   gacttggatg atgcagaaaa aatattagag aaaattgata tattgcattt ggaattcgct    120
   aagagagcag ctcctttcaa caactggttg gatggtacac gtgaagattt agtggacatg    180
   ttcattgtac acactgttga ggaaatccaa ggattgattg atgcacatgg acaatttaag    240
   gctactttgt ctgatgctga caaagagtac aactctatca ttggactggt caaagatgtt    300
   gagtcaactg tacaaaaata ccaaatacct ggtggtcttc agaacccgta cactactttg    360
   acttctagtg atttaagcaa aaaatggtct gaagtgaaac atttagtgcc ccaaagagac    420
   acgaccctcc aagctgaact cagaaaacaa caaaacaatg agatgctacg tcgtcaattt    480
   gcggagaagt caaatcaagt gggtccttgg attgagaggc aaatggacgc tgtcacggcc    540
   atcggtatgg gattgcaggt tctctggaag atcaattgca ccaactgaaa caatttaggg    600
   ctactttgtc tgatgctgac aagagtacaa ctctatcatt ggactggtca agatgttgag    660
   tcaactgtac aaaaatacca aatacctggt ggtcttcaga accngtacac tactttgact    720
   tctagtgatt taagcaaaaa atggtctgaa gtgaaacatt tagtgcccca aagagacacg    780
   accctccaag ctgaactcag aaaacaacaa aacaatgaga tgctacgtcg tcaatttgcg    840
   gagaagtcaa atcaagtggg tccttggatt gagaggcaaa tggacgctgt cacggccatc    900
   ggtatgggat tgcaaggttc tctggaagat caattgcacc aactgaaaca atacgaacag    960
   aatgtgtttg catacaagcc acatattgag gaattagaga aaatccacca agctgtacaa   1020
   gagggtatga tcttcgaaaa caggtatact caatacacaa tggagacatt acgtgttgga   1080
   tgggaacaac tattgacgtc cataaatcgc aatgtgaatg aagtagaaaa ccaaatattg   1140
   accagagact ccaaaggcat cacccaggag cag                                1173

## Description

### Introduction

Several approaches are currently available to obtain plants with increased resistance to plant insect pests. However, for plant sap-sucking insects such as aphids, planthoppers, stinkbugs, and whiteflies, only limited options are currently available to protect plants using transgenic approaches. Plants expressing *Bacillus thuringiensis* toxins targeted to Lepidopteran insects have not been shown to possess enhanced resistance towards plant sap-sucking insects (Rao et al., 1998).

dsRNA technology has been proven to be highly specific and highly effective in silencing endogenous genes in several organisms. However, the dsRNA provided to date is typically packaged in a bacterial or yeast cell or in transfection promoting agents such as liposomes. In this invention, it has now been shown that naked, unpackaged dsRNA can be used to silence genes in plant sap-sucking insects.

Plant sap-sucking insects typically feed from the sap in the vascular system of plants which they tap into with the stylets of their proboscis, causing a reduction in plant vitality and the spreading of several plant viral diseases. Plant sap-sucking insects typically have a short life-cycle and are capable of building up immense populations very quickly on a host plant.

### Background of the invention

Published PCT application WO 00/01846 generally refers to a method of alleviating pest infestation of plants, which method comprises a) identifying a DNA sequence from said pest which is critical either for its survival, growth, proliferation or reproduction, b) cloning said sequence or a fragment thereof in a suitable vector relative to one or more promoters capable of transcribing said sequence to RNA or dsRNA upon binding of an appropriate transcription factor to said promoters, and c) introducing said vector into the plant. The plant pests referred to in this published PCT application are nematodes.

US patent 6,326,193 refers to the use of recombinant insect viruses such as baculoviruses expressing dsRNA to silence selected insect genes.

Published PCT application WO 99/32619 describes generally that dsRNA may be used to reduce crop destruction by other plant pathogens such as arachnids, insects, nematodes, protozoans, bacteria, or fungi. This application shows that E. coli bacteria expressing dsRNAs can confer specific inhibitory effects on *C.* e*legans* nematode larvae that feed on these bacteria.

Timmons et al. (2001) describe that ingestion of bacteria expressing dsRNAs can produce genetic interference in the nematode *Caenorhabditis elegans.* These authors describe that from a bioengineering perspective, it may be possible to use a bioengineered dsRNA comestible to modify gene expression in any organism which has a gene-specific dsRNA response similar to that of *C. elegans.* To respond to such an intervention, that target organism would need to have both a dsRNA-response mechanism (RNAi) and a dsRNA uptake/spreading mechanism that would produce responses such as those observed in *C. elegans.*

Similarly, inhibition of gene expression after ingestion of bacteria expressing double-stranded RNA was also reported for freshwater planarian flatworms (Newmark et al., 2003).

Published PCT patent application WO 2004/005485 describes the use of vectors comprising sequences designed to control plant-parasitic nematodes by RNA interference, and transgenic plants transformed with such vectors.

Published US patent application 20030180945 generally describes chimeric genes capable of producing antisense or sense RNA equipped with a prokaryotic promoter suitable for expression of the antisense or sense RNA in a particular prokaryotic host. The prokaryotic host can be used as a source of antisense and/or sense RNA, e.g., by feeding it to an animal, such as a nematode or an insect, in which the silencing of the target gene is envisioned and monitored by reduction of the expression of the reporter gene. The target gene and reporter genes should be genes present in the cells of the target eukaryotic organism, and not in the prokaryotic host organism.

Published US patent application 20030154508 describes a method for pest control comprising exposing said pest to a compound which disrupts, within said pest, a cation-amino acid transporter/channel protein. Generally described are plant cells genetically modified to produce at least one double-stranded RNAi that is designed to be taken up by pests during feeding to block expression (or the function) of a target gene. It is described that RNAi can be used to reduce or prevent message translation in any tissue of the pest because of its ability to cross tissue and cellular boundaries. It is further described that RNAi that is contacted with a pest by soaking, injection, or consumption of a food source will cross tissue and cellular boundaries, and that RNAi can also be used as an epigenetic factor to prevent the proliferation of subsequent generations of pests.

Published PCT patent application WO 01/37654 generally describes dsRNA targeted against piercing-sucking insects and chewing insects, and that double stranded RNAs intended to confer sac-sucking insect resistance would preferentially be expressed in plant tissues on which such insects feed, e.g., primary and secondary phloem elements, and would be taken up by the insect via its sucking mechanism, e.g., its stylet. The only insect for which application of dsRNA is exemplified is *Manduca sexta,* a Lepidopteran insect. Susceptibility of this insect to RNAi was determined by treating larvae with dsRNA by either feeding or by direct injection. The results from these experiments show that with dsRNA sequences derived from three separate genes, injection into M. sexta leads to substantial decrease in expression of the endogenous gene. No results of the feeding experiment are reported.

Published PCT patent application WO 02/14472 describes methods for inhibiting target gene expression, by expressing in a cell a nucleic acid construct comprising an inverted repeat and a sense or antisense region having substantial sequence identity to a target gene, wherein the inverted repeat is unrelated to the target gene. Listed as one of the possible targets are insects such as sucking insects.

US published patent application 20030150017 describe the use of RNA molecules homologous or complementary to a nucleotide sequence of a plant pest such as nematodes and insects. Application of RNAi to a model insect species, the lepidopteran insect *Helicoverpa armigera,* in the model plant lettuce, is suggested in the Examples, but no feeding experiment is done and no results are reported.

Published PCT patent application WO 03/004644 A1 describes that the evolutionary distance between nematodes and insects is considerable, and that there is no reason to assume that while feeding dsRNA to *C. elegans* was successful, it would be a technique easily transferable to insects. It further describes the use of dsRNA technology to arthropods, and shows that direct feeding of naked, unpackaged, dsRNA failed to produce an RNAi phenotype in *Drosophila melanogaster* and *Helicoverpa armigera,* indicating that transfection promoting agents such as liposomes were necessary for effective transfection in these species. This application generally envisages that in arthropods with a simple digestive system naked dsRNA may be effective in obtaining gene silencing. No results of the delivery of naked dsRNA to any arthropod are included in this application.

Gura (2003) describes that while in *C. elegans* feeding of E. coli strains engineered to produce the double-stranded RNA can trigger RNAi, in the insect *Drosophila* the feeding of yeast cells engineered to make double-stranded RNA failed to work.

Also, Rajagopal et al. (2002) described that in *Spodoptera litura* insects, experiments to introduce dsRNA into neonate larvae of *S. litura* by soaking them in dsRNA solution or by feeding through diet were unsuccessful, since no reduction in transcript levels was detected.

Rao et al. (1998) describe that expression of a snowdrop lectin in transgenic rice plants can confer resistance to rice brown planthopper.

Hence, the prior art does not show that naked, unpackaged dsRNA or siRNA can be used to obtain gene silencing In insects through feeding.

### Summary

Provided in accordance with this invention, is a method to silence a gene of a plant sap-sucking insect, comprising applying to the diet or feed of said plant sap-sucking insect dsRNA or siRNA without transfection-promoting agents, which dsRNA or siRNA is targeted to an essential plant sap-sucking insect gene.

Also provided herein is a method of identifying gene function in a plant sap-sucking insect, comprising the step of applying dsRNA of siRNA targeting a plant sap-sucking insect gene to the diet of said insect, and evaluating phenotypic or biochemical changes in said insect, wherein said dsRNA or siRNA is applied without transfection-promoting agents.

In accordance with this invention, also provided herein is a method to obtain significant insect mortality or significant insect control by silencing of an essential gene of a plant sap-sucking insect by application of dsRNA or siRNA to the feed of said insect, as compared to control insects fed on dsRNA or siRNA targeting a non-essential gene or a gene not expressed in the plant sap-sucking insect.

In one embodiment of this invention, any of the above methods is provided, wherein said sap-sucking insect gene is homologous to a gene which when partially or completely silenced in an insect such as *Drosophila* results in a mutant with a lethal phenotype, and wherein 2 genes are homologous when they are similar in sequence to such a degree that when the two sequences are aligned, the number of positions with identical nucleotides divided by the number of nucleotides in the shorter of the sequences is higher than 70%.

In another embodiment of this invention, any of the above methods is provided, wherein said sap-sucking gene is a gene the silencing of which brings about a decreased growth, development, reproduction or survival of a plant sap-sucking insect compared to control insects fed on dsRNA or siRNA targeting a gene not expressed in the plant sap-sucking insect.

In a further embodiment any of the above methods is provided, wherein said dsRNA or siRNA does not silence genes of a plant, or of non-target animals, such as plant sap-sucking insect predators or animals such as reptiles, amphibians, birds, or mammals.

Further provided herein are any of the above methods, wherein a portion of a target sequence is selected which is present in several plant sap-sucking insects of a plant host in identical sequence or with high sequence identity, wherein a sequence with high sequence identity refers to the number of positions with identical nucleotides divided by the number of nucleotides in the shorter of the sequences, being higher than 95 %.

Also provided herein are any of the above methods, wherein said sap-sucking insect gene is selected from the group consisting of: the eucaryotic translation initiation factor 1A (elF1A), an (alpha) tubulin gene, an alpha-actinin gene, and an ecdysone receptor gene.

Further provided herein are any of the above methods, wherein said plant sap-sucking insect genes are those plant sap-sucking insect genes identified using the primers of any one of SEQ ID NO:1-4 and SEQ ID NO:9 and 10, or plant sap-sucking insect genes corresponding to or comprising any one of the sequences of SEQ ID NO:5 to 8, SEQ ID NO:11 or SEQ ID NO:12, as well as aphid genes homologous to the sequences of SEQ ID NO:5 to 8, SEQ ID NO:11 or SEQ ID NO:12, wherein 2 genes are homologous when they are similar in sequence to such a degree that when the two sequences are aligned, the number of positions with identical nucleotides divided by the number of nucleotides in the shorter of the sequences is higher than 70%, as well as such methods, wherein the dsRNA or siRNA targets the *A. gossypii* elF1A sequence of SEQ ID NO: 5 only in the portion from nucleotide position 72 to the end in SEQ ID NO:5.

Also provided herein is any of the above methods, wherein said plant sap-sucking insect gene comprises the sequence of any one of SEQ ID NO:5 to 8, or SEQ ID NO:12. Further provided herein is a method of identification of novel targets for insecticidal compounds, comprising the steps of: a) applying dsRNA or siRNA without transfection-promoting agents to the feed or diet of a plant-sap sucking insect; b) analyzing which genes when silenced confer lethality to said insect, c) cloning and characterizing said genes thus analyzed; d) identifying compounds that disrupt or inactivate said gene or the RNA or protein encoded thereby; and e) contacting said compounds with said insect or feed or diet of said insect to confirm the pesticidal nature of said compound.

### Detailed Description of the invention

In accordance with the invention, dsRNA or siRNA is fed to a plant sap-sucking insect without being contained in a cell or a transfection-promoting agent. In one embodiment of the invention, this feeding is on a plant which expresses the dsRNA or siRNA so that it enters the sap contained in its vascular system. A "transfection promoting agent", as used herein, refers to a lipid-containing material that allows or enhances passage of the cell membrane and hence secures uptake by a cell of an extracellular molecule or compound such as a dsRNA, particularly liposomes. Such agents are described in published PCT patent application WO 03/004644. For the avoidance of doubt, the cationic oligopeptides used in some embodiments of the current invention are not included in this definition of transfection-promoting agents. A dsRNA or siRNA delivered without transfection promoting agent is also referred to herein as "naked" and/or "unpackaged" dsRNA or siRNA. A cationic oligopeptide, as used herein, does not "package" dsRNA or siRNA (contrary to a transfection promoting agent, such as a liposome) and hence can be supplied together with the dsRNA of siRNA in the case of "unpackaged" delivery of dsRNA or siRNA. The term "chimeric" when referring to a gene or DNA sequence is used to refer to a gene or DNA sequence comprising at least two functionally relevant DNA fragments (such as promoter, 5'UTR, coding region, 3'UTR, intron) that are not naturally associated with each other and/or originate, for example, from different sources. "Foreign" referring to a gene or DNA sequence with respect to a plant species is used to indicate that the gene or DNA sequence is not naturally found in that plant, or is not naturally found in that genetic locus in that plant. The term "foreign DNA" will be used herein to refer to a DNA sequence as it has incorporated into the genome of a plant as a result of transformation.

Two sequences or genes (or parts thereof) which are "homologous" or "similar", as used herein, are similar in sequence to such a degree that when the two sequences are aligned, the percent sequence identity, *i.e.*, the number of positions with identical nucleotides divided by the number of nucleotides in the shorter of the sequences, is higher than 70%, higher than 85%, higher than 90%, higher than 95%, or is between 96 % and 100 %. Homologous genes or parts thereof, as used herein, do not require any evolutionary relationship, though genes related to each other by independent evolution from the same ancestral gene can be homologous genes as used herein. As used herein, a gene "homolog" (or homologous gene) can be a gene paralog (or a paralogous gene) and a gene ortholog (or an orthologous gene). In one embodiment of the invention a homologous gene is an orthologous gene (i.e., a similar gene in a different species likely evolved from a common ancestor and which normally has retained essentially the same or the same function). Sequences or parts of sequences which have "high sequence identity", as used herein, refers to the number of positions with identical nucleotides divided by the number of nucleotides in the shorter of the sequences, being higher than higher than 95%, or between 96 % and 100 %. A target gene, or at least a part thereof, as used herein, preferably has high sequence identity to the dsRNA of the invention in order for efficient gene silencing to take place in the target pest. Identity in sequence of the dsRNA or siRNA with a part of the target gene RNA is included in the current invention but is not necessary.

For the purpose of this invention, the "sequence identity" of two related nucleotide or amino acid sequences, expressed as a percentage, refers to the number of positions in the two optimally aligned sequences which have identical residues (x100) divided by the number of positions compared. A gap, i.e., a position in an alignment where a residue is present in one sequence but not in the other is regarded as a position with non-identical residues. The alignment of the two sequences is performed by the Needleman and Wunsch algorithm (Needleman and Wunsch 1970). A computer-assisted sequence alignment can be conveniently performed using a standard software program such as GAP which is part of the Wisconsin Package Version 10.1 (Genetics Computer Group, Madison, Wisconsin, USA) using the default scoring matrix with a gap creation penalty of 50 and a gap extension penalty of 3.

For the purpose of the invention, the "complement of a nucleotide sequence X" is the nucleotide sequence which would be capable of forming a double stranded DNA molecule with the represented nucleotide sequence, and which can be derived from the represented nucleotide sequence by replacing the nucleotides by their complementary nucleotide according to Chargaff's rules (A<>T; G<>C) and reading in the 5' to 3' direction, i.e., in opposite direction of the represented nucleotide sequence.

As used herein, "dsRNA" refers to double-stranded RNA that comprises a sense and an antisense portion of a selected target gene (or sequences with high sequence identity thereto so that gene silencing can occur), as well as any smaller double-stranded RNAs formed therefrom by RNAse or dicer activity. Such dsRNA can include portions of single-stranded RNA, but contains at least 19 nucleotides double-stranded RNA. In one embodiment of the invention, the dsRNA is a hairpin RNA which contains a loop or spacer sequence between the sense and antisense sequences of the gene targeted, preferably such hairpin RNA spacer region contains an intron, particularly the rolA gene intron (Pandolfini et al., 2003), the dual orientation introns from pHellsgate 11 or 12 (see WO 02/059294, and SEQ ID NO: 25 and 15 therein) or the pdk intron (Flaveria trinervia pyruvate orthophosphate dikinase intron 2 ; see WO99/53050). "siRNAs" as used herein are small interfering (double-stranded) RNA molecules of 16-30 bp, 19-28 bp, or 21-26 bp, e.g., the RNA forms that can be created by RNAseIII or dicer activity from longer dsRNA. "siRNAs" as used herein include any double-stranded RNA of 19 to 26, or 21 to 24 basepairs that can interfere with gene expression when present in a cell wherein such gene is expressed. siRNA can be synthetically made, expressed and secreted directly from a transformed cell or can be generated from a longer dsRNA by enzymatic activity. These siRNAs can be blunt-ended or can have overlapping ends.

In one embodiment of the invention, sense and antisense RNAs can be separately expressed in vitro or in host cells, e.g., in cells of a plant from different chimeric gene constructs using the same or a different promoter or from a construct containing two convergent promoters in opposite orientation. These sense and antisense RNAs which are formed, e.g., in the same plant cells, can then combine to form dsRNA or siRNA. It is clear that whenever reference is made herein to a dsRNA or siRNA chimeric gene or a dsRNA or siRNA molecule, that such dsRNA or siRNA formed, e.g., in plant cells, from sense and antisense RNA produced separately is also included. Also synthetically made siRNA or dsRNA annealing RNA strands are included herein when the sense and antisense strands are present together.

A dsRNA or siRNA "targeting" a plant sap-sucking insect gene, as used herein, refers to a dsRNA or siRNA that is designed to be identical or have high sequence identity to an endogenous plant sap-sucking insect gene (a target gene), and as such is designed to silence such gene upon application to such insect. One dsRNA can target one or several homologous genes in one plant sap-sucking insect or one or several homologous genes in different plant sap-sucking insects which can feed on the same host plant.

The dsRNA chimeric gene, encoding a dsRNA targeting a plant sap-sucking essential gene, can be stably inserted in a conventional manner into the genome of a single plant cell, and the so-transformed plant cell can be used in a conventional manner to produce a transformed plant that has increased insect resistance. In this regard, a disarmed Ti-plasmid, containing the dsRNA chimeric gene, in *Agrobacterium tumefaciens* can be used to transform the plant cell, and thereafter, a transformed plant can be regenerated from the transformed plant cell using the procedures described in the art, for example, in EP 0 116 718, EP 0 270 822, PCT publication WO 84/02913 and published European Patent application ("EP") 0 242 246. Preferred Ti-plasmid vectors each contain the dsRNA chimeric gene between the border sequences, or at least located to the left of the right border sequence, of the T-DNA of the Ti-plasmid. Of course, other types of vectors can be used to transform the plant cell, using procedures such as direct gene transfer (as described, for example in EP 0 233 247), pollen mediated transformation (as described, for example in EP 0 270 356, PCT publication WO 85/01856, and US Patent 4,684,611), plant RNA virus-mediated transformation (as described, for example in EP 0 067 553 and US Patent 4,407,956), liposome-mediated transformation (as described, for example in US Patent 4,536,475), and other methods such as the methods for transforming certain lines of corn (e.g., US patent 6,140,553; Fromm et al., 1990; Gordon-Kamm et al., 1990) and rice (Shimamoto et al., 1989; Datta et al., 1990) and the method for transforming monocots generally (PCT publication WO 92/09696). For cotton transformation, especially preferred is the method described in PCT patent publication WO 00/71733. For soybean transformation, reference is made to methods known in the art, e.g., Hinchee et al. (1988) and Christou et al. (1990) or the method of WO 00/42207.

The resulting transformed plant can be used in a conventional plant breeding scheme to produce more transformed plants with the same characteristics or to introduce the dsRNA gene in other varieties of the same or related plant species. Seeds, which are obtained from the transformed plants, contain the dsRNA gene as a stable genomic insert. Plants comprising a dsRNA or siRNA in accordance with the invention include plants comprising or derived from root stocks of plants comprising the dsRNA chimeric gene of the invention, e.g., fruit trees. Hence, any non-transgenic grafted plant parts inserted on a transformed plant or plant part are included in the invention since the RNA interference signal is transported to these grafted parts and any aphids feeding on such grafted plant are similarly affected by the dsRNA or siRNA of the invention.

A DNA encoding a dsRNA is inserted in a plant cell genome so that this DNA is downstream (i.e., 3') of, and operably linked to, a plant-expressible promoter which can direct expression in plant cells. This is preferably accomplished by inserting the dsRNA chimeric gene in the plant cell genome, particularly in the nuclear or plastid (e.g., chloroplast) genome.

A 'plant-expressible promoter' as used herein refers to a promoter that ensures expression of a dsRNA of the invention in a plant cell. Examples of promoters directing constitutive expression in plants are known in the art and include: the strong constitutive 35S promoters (the "35S promoters") of the cauliflower mosaic virus (CaMV), e.g., of isolates CM 1841 (Gardner et al., 1981), CabbB-S (Franck et al., 1980) and CabbB-JI (Hull and Howell, 1987); promoters from the ubiquitin family (e.g., the maize ubiquitin promoter of Christensen et al., 1992, see also Comejo et al., 1993), the gos2 promoter (de Pater et al., 1992), the emu promoter (Last et al., 1990), actin promoters such as the promoter described by An et al. (1996), the rice actin promoter described by Zhang et al. (1991); promoters of the Cassava vein mosaic virus (WO 97/48819, Verdaguer et al. (1998)), the pPLEX series of promoters from Subterranean Clover Stunt Virus (WO 96/06932, particularly the S4 or S7 promoter), a alcohol dehydrogenase promoter, e.g., pAdh1S (GenBank accession numbers X04049, X00581), and the TR1' promoter and the TR2' promoter (the "TR1' promoter" and "TR2' promoter", respectively) which drive the expression of the 1' and 2' genes, respectively, of the T-DNA (Velten et al., 1984). Alternatively, a plant-expressible promoter can be a tissue-specific promoter, i.e., a promoter directing a higher level of expression in some cells or tissues of the plant, e.g., in green tissues (such as the promoter of the PEP carboxylase). The plant PEP carboxylase promoter (Pathirana et al., 1997) has been described to be a strong promoter for expression in vascular tissue and is useful in one embodiment of the current invention. Alternatively, a plant-expressible promoter can also be a wound-inducible promoter, such as the promoter of the pea cell wall invertase gene (Zhang et al., 1996). A 'wound-inducible' promoter as used herein means that upon wounding of the plant, either mechanically or by insect feeding, typically by piercing of the plant to access the vascular system in the plant sap-sucking insects, expression of the coding sequence under control of the promoter is significantly increased in such plant. It has been shown that plant sap-sucking insects can also cause plant defense responses similar to those observed for other pathogens and wounding (Moran and Thompson, 2001). Promoters of certain of such genes induced by plant sap-sucking insects, preferably when their expression is preferentially in the vascular tissue, particularly phloem, can also be used in accordance with the current invention.

In one embodiment of this invention the plant-expressed promoter is a vascular-specific promoter such as a phloem-specific promoter. A "vascular-specific" promoter, as used herein, is a promoter which is at least expressed in vascular cells, or a promoter which is preferentially expressed in vascular cells. Expression of a vascular-specific promoter need not be exclusively in vascular cells, expression in other cell types or tissues is possible. A "phloem-specific promoter" as used herein, is a plant-expressible promoter which is at least expressed in phloem cells, or a promoter which is preferentially expressed in phloem cells. Expression of a phloem-specific promoter need not be exclusively in phloem cells, expression in other cell types or tissues, e.g., xylem tissue, is possible. In one embodiment of this invention, a phloem-specfic promoter is a plant-expressible promoter at least expressed in phloem cells, wherein the expression in non-phloem cells is more limited (or absent) compared to the expression in phloem cells. Examples of suitable vascular-specific or phloem-specific promoters in accordance with this invention include but are not limited to the promoters selected from the group consisting of: the SCSV3, SCSV4, SCSV5, and SCSV7 promoters (Schünmann et al., 2003), the rolC gene promoter of *Agrobacterium rhizogenes* (Kiyokawa et al., 1994; Pandolfini et al., 2003; Graham et al., 1997; Guivarc'h et al., 1996, Almon et al;, 1997), the rolA gene promoter of *Agrobacterium rhizogenes* (Dehio et al., 1993), the promoter of the *Agrobacterium tumefaciens* T-DNA gene 5 (Korber et al. 1991), the rice sucrose synthase RSs1 gene promoter (Shi et al., 1994), the CoYMV or Commelina yellow mottle badnavirus promoter (Medberry et al., 1992; Zhou et al., 1998), the CFDV or coconut foliar decay virus promoter (Rohde et al., 1994; Hehn and Rhode, 1998), the RTBV or rice tungro bacilliform virus promoter (Yin and Beachy, 1995; Yin et al., 1997), the pea glutamine synthase GS3A gene (Edwards et al., 1990; Brears et al., 1991), the invCD111 and invCD141 promoters of the potato invertase genes (Hedley et al., 2000), the promoter isolated from Arabidopsis shown to have phloem-specific expression in tobacco by Kertbundit et al (1991), the VAHOX1 promoter region (Tornero et al., 1996), the pea cell wall invertase gene promoter (Zhang et al., 1996), the promoter of the endogenous cotton protein related to chitinase of US published patent application 20030106097, an acid invertase gene promoter from carrot (Ramloch-Lorenz et al., 1993), the promoter of the sulfate transporter gene Sultr1;3 (Yoshimoto et al., 2003), a promoter of a sucrose synthase gene (Nolte and Koch, 1993), and the promoter of a tobacco sucrose transporter gene (Kuhn et al., 1997). Also, any promoter homologous to (or having a high sequence identity to) any of the above promoters and also exhibiting phloem-specific expression, as defined herein, can be used. The selection of a particular promoter can depend on the insect species mainly targeted (e.g., a specific insect targeted can be mostly a leaf feeder or mostly a root feeder, allowing different promoter specificities to be used), and on the expression level and tissue distribution desired. These promoters can be combined with enhancer elements, they can be combined with minimal promoter elements, or can comprise repeated elements to ensure the expression profile desired.

Elements which can be used to increase expression in plant cells can be: introns at the 5' end or 3' end of the chimeric gene, e.g., the hsp70 intron, promoter enhancer elements, duplicated or triplicated promoter regions, 5' leader sequences different from another transgene or different from an endogenous (plant host) gene leader sequence, 3' trailer sequences different from another transgene used in the same plant or different from an endogenous (plant host) trailer sequence.

The dsRNA gene of the invention can be inserted in the plant genome so that the inserted gene part is upstream (i.e., 5') of suitable 3' end transcription regulation signals (i.e., transcript formation and polyadenylation signals). This is preferably accomplished by inserting the dsRNA chimeric gene in the plant cell genome. Preferred polyadenylation and transcript formation signals include those of the nopaline synthase gene (Depicker et al., 1982), the octopine synthase gene (Gielen et al., 1984), the SCSV or the Malic enzyme terminators (Schunmann et al., 2003), and the T-DNA gene 7 (Velten and Schell, 1985), which act as 3'-untranslated DNA sequences in transformed plant cells.

The dsRNA chimeric gene can optionally be inserted in the plant genome as a hybrid gene, containing several dsRNA regions which target different genes in the same or different plant sap-sucking insects, or which target different portions of the same gene. Also, it is convenient to include in the transforming DNA of the invention also a selectable or scorable marker gene, such as the bar or the neo gene, so that transformed plants can easily be selected by application of glufosinate or kanamycin, respectively, as is well known in the art.

Although plant delivery of a dsRNA or siRNA is an embodiment of this invention, in accordance with this invention, application of the dsRNA or siRNA of the invention to a plant sap-sucking insect can be done in several ways, and need not be by way of a plant expressing a dsRNA or siRNA. Any method of delivery of siRNA or dsRNA not contained in a cell or a cell-transfection agent (such as a liposome) is included herein, e.g., *in vitro* produced siRNA or dsRNA applied to an insect diet or feed.

"Insecticidal activity" of a dsRNA or siRNA, as used herein, refers to the capacity to obtain mortality in insects when such RNA is fed to insects, preferably by expression in a recombinant host such as a plant, which mortality is significantly higher than the controls (using a non-insect dsRNA or buffer). "Insect-control" of a dsRNA or siRNA, as used herein, refers to an amount of RNA which is sufficient to limit damage on a plant by insects feeding on such plant, e.g., by killing the insects or by inhibiting the insect development, fertility or growth in such a manner that they provide less damage to a plant, produce fewer offspring, are less fit or more susceptible to predator attack, or that insects are even deterred from feeding on such plant.

Information on how to design optimal dsRNA or siRNA sequences once a target gene is known can be found with commercial providers, e.g., the companies Ambion and Cenix BioScience (Ambion Inc., 2130 Woodward Street, Austin, TX 78744-1832, USA; and see www.ambion.com; and Cenix BioScience GmbH, Pfotenhauerstr. 108, 01307 Dresden, Germany, see www.cenix-bioscience.com). Preferably, the dsRNAs or siRNAs to be used in this invention target at least one essential plant sap-sucking insect gene, or an essential plant sap-sucking insect gene occurring without significant sequence divergence (at least in a certain region) in a range of plant sap-sucking insect pests of the host plant concerned. In one embodiment of this invention, such dsRNAs or siRNAs do not silence genes of the plant host or of other non-target animals, such as plant sap-sucking insect predators (e.g., ladybird larvae, anthocorid bugs, lacewing, parasitic wasps or hoverfly larvae) or animals such as reptiles, amphibians, birds, or mammals. This can be analyzed in available databases, e.g., by a BLAST search (see also www.ncbi.nlm.nih.gov/BLAST) or by hybridization with existing DNA libraries of representative non-target organisms. In this respect, when using the *A. gossypii* elF1A sequence of SEQ ID NO: 5 to transform a plant with a dsRNA chimeric gene, preferably only that portion from nucleotide position 72 to the end in SEQ ID NO:5 should be used as gene target in designing the dsRNA molecule, or at least the portion from nucleotide position 50 to 73 in SEQ ID NO:5 should be avoided in the dsRNA. In one embodiment, a portion of a target sequence is selected which is present in several plant sap-sucking insects of a plant host in identical sequence or with high sequence identity, e.g., a part of a class of gene(s) with high sequence homology in several plant sap-sucking insect pests.

A plant sap-sucking insect "target gene", "an essential gene", or "a gene essential for a plant sap-sucking insect", as used herein, is a gene the silencing of which brings about a decreased growth, development, reproduction or survival of a plant sap-sucking insect. In one embodiment, the partial or complete silencing of an essential gene of a plant sap-sucking insect results in significant insect mortality or significant insect control when such gene is silenced by dsRNA or siRNA compared to control insects fed on dsRNA or siRNA targeting a non-essential gene or a gene not expressed in the plant sap-sucking insect. In one embodiment of this invention, the dsRNA or siRNA of the invention corresponds to an exon in the target gene.

In one embodiment of the invention genes expressed in cells of the plant sap-sucking insect gut tissue or in the midgut are targeted, preferably genes involved in gut cell metabolism, growth or differentiation. These genes can encode plant sap-sucking insect gut membrane proteins such as transporter molecules or ion pumps, e.g., a vATPase (e.g., a homolog to the *Drosophila* gene of Genbank accession number AF143200) or an amino acid transporter gene. Useful plant sap-sucking insect target genes in accordance with the invention also include genes encoding the following: a transcription factor; a peptidylglycine alpha-amidating monooxygenase (e.g., a homolog of the Drosophila phm gene described in Jiang et al., 2000); a cystein protease (Cristofoletti et al., 2003), an aminopeptidase (e.g., a gene homologous to a aminopeptidase N gene expressed in the gut of *Drosophila,* or a gene encoding the pea aphid aminopeptidase N (Rahbé et al. ,1995; Cristofoletti et al., 2003), a dipeptidase, a sucrase/ transglucosidase (see, Ashford et al. 2000; Cristofoletti et al. (2003)); a translation initiation factor (such as the eucaryotic translation initiation factor 1A (elF1A) (e.g., based on the homolog in Drosophila (Myrick and Dearolf, 2000, Genbank accession number AF169359)), a translation elongation factor (such as a plant sap-sucking insect gene homologous to the *Drosophila* elongation factor 1 alpha gene (Hovemann et al., 1988, Genbank accession number X06869)); a splicing factor (such as a gene homolog of the

Drosophila SF1 gene (Marzoui et al., 1999), or Genbank accession number NM_079915); an IAP inhibiting apoptosis (e.g., a homolog of the IAP gene described in Hay, 2000 or Genbank accession number AA801628); an (alpha) tubulin (e.g., the homolog of the Drosophila alpha-tubulin gene decribed in Matthews and Kaufman, 1987 or in Genbank accession number Al 124284), an actin or alpha-actinin (e.g., the homolog to the Drosophila gene described in Fyrberg et al., 1998; Dubreuil and Wang, 2000, and in Genbank accession number NM_058137); a histone protein (e.g., a homolog of the H2A.F/Z family of Drosophila histone genes (Clarkson and Saint, 1999), such as H2AvD (van Daal and Elgin, 1992, Clarkson et al., 1999; Genbank accession number NM_079795); a histone deacetylase (such as a homolog of the histone deacetylases HDAC1 (Mottus et al., 2000); HDAC3 (Johnson et al, 1998) or HDAC4a (Zeremsky et al., 2003), Genbank accession number AF538713); a cell cycle protein; a protein essential for cellular respiration; a receptor for an insect-specific hormonal signal, a juvenile hormone receptor (e.g., a plant sap-sucking juvenile hormone receptor gene described in published PCT patent applications WO99/36520 and WO01/02436), an insect peptidic hormone receptor; plant sap-sucking insect homologous genes of the dmHelicase, dmPITP or dmSPL genes of *Drosophila* identified in WO01/42479; a (part of) ecdysone receptor (e.g., a plant sap-sucking ecdysone receptor gene as the *M. persicae* or *B. tabaci* genes described in published PCT patent applications WO99/36520 and WO01/02436; or an insect gene homologous to the Drosophila ecdyson receptor gene (see, e.g., the ecdyson receptor gene forms described in Bender et al. (1997); Lam and Thummel, 2000), or to the Drosophila ultraspiracle gene (Henrich et al., 2000)); a protein essential for regulating ion balance in the cells (e.g., a proton-pump, a NA/K pump, etc.); an intestinal protease; etc.. Possible target genes are also other genes encoding enzymes involved in sucrose metabolism in the plant sap-sucking insect, preferably in the gut, or genes encoding digestive enzymes such as the trypsin-like protease and the cathepsin B-like protease that were recently described in a Homopteran plant sap-sucking insect, the rice brown planthopper (Foissac et al., 2002), and homologous genes found in other plant sap-sucking insects.

Preferred target sequences in accordance with this invention are the plant sap-sucking insect genes identified using the primers of any one of SEQ ID NO:1-4 and SEQ ID NO:9 and 10, or plant sap-sucking insect genes corresponding to or comprising any one of the sequences of SEQ ID NO:5 to 8, SEQ ID NO:11 or SEQ ID NO:12, as well as aphid genes homologous to or having high sequence identity with the sequences of SEQ ID NO:5 to 8, SEQ ID NO:11 or SEQ ID NO:12, this includes other portions of the same aphid genes or genes of other plant sap-sucking insects having high sequence identity or homology to the sequences of SEQ ID NO:5 to 8, SEQ ID NO:11 or SEQ ID NO:12. It is preferred that only portions of the target gene which are known, i.e., those portions of SEQ ID NO:5 to 8, SEQ ID NO:11 or SEQ ID NO:12 that have no "n" positions, are used in the design of the chimeric genes of the invention.

In one embodiment of this invention, target genes are plant sap-sucking insect genes homologous to a gene which when partially or completely silenced (or otherwise prevented to express a functional protein or RNA) in an insect, e.g., *Drosophila,* results in a mutant with a lethal phenotype (see, e.g., www.fruitfly.org/p_disrupt/; Spradling et al., 1999; and Adams and Sekelsky, 2002 and references cited therein), particularly when such gene is expressed in insect gut cells, particularly in the gut cells lining the gut lumen, especially gut cells lining the midgut. Homologs of Drosophila genes are easily found by existing techniques, e.g., PCR amplification of the plant sap-sucking insect homologous gene using primers targeting a *Drosophila* essential gene (e.g., in cDNA or genomic libraries of a preferred target insect), or by routine similarity searches in available DNA sequence databases of plant sap-sucking insects.

Target genes can also be found using similar sequences of essential insect genes isolated and characterized in other non-*Drosophila* insects. In one embodiment of the invention, the target gene preferably produces a stable mRNA in the aphid. Also, in one embodiment of the invention, although the target gene has to be transcribed in the target insect, for obtaining an optimal silencing effect, it is preferred that the target gene does not produce abundant amounts of RNA.

To test performance of a certain dsRNA or siRNA in plants, the system as described in published PCT application WO 03/052108 can be used, wherein plants produce dsRNA/siRNA and effects on the aphids growing on such plants can be assessed. As a control, a different non-essential gene that is absent in aphids, such as a gene encoding a *gfp* (green fluorescent protein)-specific dsRNA, is tested in parallel in the same viral construct.

Also, in the dsRNA chimeric gene of the invention a nuclear localization signal can be added as described in published US patent application 20030180945.

As used herein, nucleotide sequences of RNA molecules may be identified by reference to DNA nucleotide sequences of the sequence listing. However, the person skilled in the art will understand whether RNA or DNA is meant depending on the context. Furthermore, the nucleotide sequence is identical except that the T-base is replaced by uracil (U) in RNA molecules.

The length of the first (e.g., sense) and second (e.g., antisense) nucleotide sequences of the dsRNA molecules of the invention may vary from about 10 nucleotides (nt) up to a length equaling the length in nucleotides of the transcript of the target gene. The length of the first or second nucleotide sequence of the dsRNA of the invention can be at least 15 nt, or at least about 20 nt, or at least about 50 nt, or at least about 100 nt, or at least about 150 nt, or at least about 200 nt, or at least about 500 nt, or at least about 1600 nt. If not all nucleotides in a target gene sequence are known, it is preferred to use such portion for which the sequence is known and which meets other beneficial requirements of the invention.

It will be appreciated that the longer the total length of the first nucleotide sequence in the dsRNA of the invention is, the less stringent the requirements for sequence identity between the total sense nucleotide sequence and the corresponding sequence in the target gene becomes. The total first nucleotide sequence can have a sequence identity of at least about 75% with the corresponding target sequence, but higher sequence identity can also be used such as at least about 80 %, at least about 85%, at least about 90%, at least about 95%, about 100%. The first nucleotide sequence can also be identical to the corresponding part of the target gene. However, it is advised that the first nucleotide sequence includes a sequence of 19 or 20, or about 19 or about 20 consecutive nucleotides, or even of about 50 consecutive nucleotides, or about consecutive 100 nucleotides, or about 150 consecutive nucleotides with only one mismatch, preferably with 100% sequence identity, to the corresponding part of the target gene. For calculating the sequence identity and designing the corresponding first nucleotide sequence, the number of gaps should be minimized, particularly for the shorter sense sequences..

The length of the second (e.g., antisense) nucleotide sequence in the dsRNA of the invention is largely determined by the length of the first (e.g., sense) nucleotide sequence, and may correspond to the length of the latter sequence. However, it is possible to use an antisense sequence which differs in length by about 10% without any difficulties. Similarly, the nucleotide sequence of the antisense region is largely determined by the nucleotide sequence of the sense region, and may be identical to the complement of the nucleotide sequence of the sense region. Particularly with longer antisense regions, it is however possible to use antisense sequences with lower sequence identity to the complement of the sense nucleotide sequence, such as at least about 75% sequence identity, or least about 80%, or at least about 85%, more particularly with at least about 90% sequence identity, or at least about 95% sequence to the complement of the sense nucleotide sequence. Nevertheless, it is advised that the antisense nucleotide sequence always includes a sequence of 19 or 20, about 19 or about 20 consecutive nucleotides, although longer stretches of consecutive nucleotides such as about 50 nucleotide, or about 100 nucleotides, or about 150 nucleotides with no more than one mismatch, preferably with 100% sequence identity, to the complement of a corresponding part of the sense nucleotide sequence can also be used. Again, the number of gaps should be minimized, particularly for the shorter (19 to 50 nucleotides) antisense sequences.

In one embodiment of the invention, the DNA molecules according to the invention may comprise a DNA region encoding a spacer between the DNA region encoding the first and second nucleotide sequences. As indicated in WO 99/53050 the spacer may contain an intron to enhance gene silencing. A particularly preferred intron functional in cells of plants is the pdk intron (Flaveria trinervia pyruvate orthophosphate dikinase intron 2; see WO99/53050), the delta 12 desaturase intron from *Arabidopsis* (Smith et al., 2000) or the intron of the rolA gene (Magrelli et al., 1994; Spena and Langenkemper, 1997).

In one embodiment of the invention, the dsRNA molecule may further comprise one or more regions having at least 94% sequence identity to regions of at least 19 consecutive nucleotides from the sense nucleotide sequence of the target gene, different from the at least 19 consecutive nucleotides as defined in the first region, and one or more regions having at least 94% sequence identity to at least 19 consecutive nucleotides from the complement of the sense nucleotide sequence of the target gene, different from the at least 19 consecutive nucleotides as defined in the second region, wherein these additional regions can basepair amongst themselves.

Plants to which the current invention can be applied include but are not limited to the following plants: corn, cotton, rice, soybean, Brassica species plants, Brassica napus, cauliflower, carrot, pea, wheat, barley, rye, tomato, potato, sugarbeet, cut flowers, roses, fruit plants (apple, pear, peach, strawberry, etc.), trees (such as poplar and willow), and lettuce. In one embodiment of the invention, the plants to which the invention is applied are cotton or rice plants. When cotton is the host plant of the invention the dsRNA preferably targets genes in one or all of the following sucking insects: Aphis gossypil, Myzus persicae, Lygus bugs, whitefly, stink bugs, thrips, and Creontiades dilutus, particularly Aphis gossypii, Myzus persicae and Creontiades dilutus.

Together with the strategy of this invention, it is preferred to use other tactics for aphid control, such as expression of a snowdrop (*Galanthus nivalis*) lectin as described by Down et al. (1996), Stoger et al. (1999) and Rao et al. (1998) or the mannose-binding lectins of Roy et al. (2002) in plants, preferably in the phloem of plants and/or the expression of a protease inhibitor such as the soybean Kunitz trypsin inhibitor (Foissac et al., 2002), or the variant mustard trypsin inhibitor Chy8 (Ceci et al., 2003) and similar protease inhibitors active against plant sap-sucking insects, particularly aphids. Also the timely application of effective chemical or biological insecticides, and the use of natural aphid predators are possible tactics to use. Also, existing endogenous resistance genes such as the tomato *Mi* nematode-resistance gene (which also confers resistance to certain aphid species, see Rossi et al., 1998) or the VAT gene (WO 2004072109) can be used to protect a plant against aphids and provide different mechanisms of resistance, hence minimizing chances of insect resistance development.

Also a plant can co-express antibacterial proteins or peptides, preferably in the phloem, to kill or negatively affect symbiontic bacteria occurring in aphids that are believed to provide the aphids with certain essential compounds (such as amino acids) which they may not get sufficiently from feeding on plant sap. These proteins can be any one of the antibacterial peptides or proteins known in the art which are effective in inhibiting bacterial growth, and which are preferably provided with a signal for extracellular or for phloem targeting. Such a protein signal sequence can be found in many proteins which are targeted to the phloem, e.g., the GNA snowdrop lectin described above. Also, a dsRNA can be expressed in the plants of the invention, which dsRNA targets an essential gene of such symbiontic bacteria, e.g., the essential genes described by Shigenobu et al. (2000) for the genome of the bacterial symbiont (Buchnera sp.) of the pea aphid, and homologous forms for symbionts in other aphids.

Together with the dsRNA or siRNAs of the current invention, a chimeric gene encoding a cationic oligopeptide can be expressed in plants. With a "cationic oligopeptide" as used herein, is meant an oligopeptide of larger then 5 and smaller then 40 amino acids with a net positive charge, and the ability to facilitate transport of siRNA or dsRNA across an insect cell membrane. Such cationic oligopeptides are preferably between 5 and 40 amino acids long, between 10 and 30 amino acids long, particularly of about 12-18 amino acids long, more particularly 12-18 amino acids long, and can bind to the dsRNA of the invention and besides stabilizing the dsRNA, they facilitate the entry of the dsRNA into the sap-sucking insect cells, particularly their gut cells, preferably their midgut cells. In this embodiment, such a cationic oligopeptide, a sequence of multiple such cationic peptides separated by spacer amino acids which are cleavable inside or outside the cell, or a cationic oligopeptide fused to a phloem-targeting signal or to another protein (such as the prosystemin fusion described by Tortiglione et al. (2003)) is expressed in the same cells as the dsRNA or siRNA of the invention, preferably but not necessarily using the same promoter (in different chimeric genes), leading to accumulation of the peptide and the dsRNA of the invention in the plant cells and/or phloem. In one embodiment of this invention, the cationic oligopeptide is a poly-Arginine 12-mer (Unnamalai et al., 2004). Other cationic oligopeptides that can be co-expressed in any plant include from 5 to 40, 10 to 30, or 12-18 amino acids long poly-Arginine, poly-Lysine or poly-Histidine peptides or mixtures of any of these 3 basic amino acids, or the penetratin peptide, transportan peptide, TAT peptide, MAP peptide, R7 peptide, pVEC peptide, MPG-delta-NLS peptide, KALA peptide or Buforin 2 peptide, as described in Järver and Langel (2004), or other oligopeptides smaller then 40 amino acids with a net positive charge, and the ability to facilitate transport of siRNA or dsRNA across an insect cell membrane, preferably the MPG-delta-NLS peptide, the 12-amino acids poly-Arginine peptide or the TAT peptide. The chimeric gene encoding the dsRNA and the chimeric gene encoding the cationic oligopeptide can be assembled in one transforming DNA, e.g., in one T-DNA insert in an *Agrobacterium* plasmid, to secure expression from one locus in the plant. These cationic oligopeptides are useful for facilitating transfer of any dsRNA or siRNA into the cells of any insect species, not just the sap-sucking insects. Such insects include insects used as model species, or insects pests of com, cotton, rice, soybean, Brassica species plants, oilseed rape, cabbage, cauliflower, carrot, pea, wheat, barley, rye, tomato, potato, sugarbeet, cut flowers, roses, fruit plants (apple, pear, peach, strawberry, etc.), trees (such as poplar and willow), and lettuce, particularly the european corn borer, cotton bollworms, and corn rootworms, besides the plant sap-sucking insect pests described herein. Particularly such insects are selected from the list consisting of: *Drosophila melanogaster, Anopheles* spp. insects, *Helicoverpa zea, Helicoverpa armigera, Helicoverpa punctigera, Heliothis virescens, Ostrinia nubilalis, Spodoptera frugiperda, Agrotis ipsilon, Pectinophora gossypiella, Scirphophaga incertulas, Cnaphalocrocis medinalis, Sesamia inferens, Chilo partellus, Anticarsia gemmatalis, Plathypena scabra, Pseudoplusia includens, Spodoptera exigua, Spodoptera omithogalli, Epinotia aporema, Rachiplusia nu, Chilo suppressalis, Scirpophaga incertu*/*as, Sesamia inferens, Cnaphalocrocis medinalis, Hereitogramma licarisa*/*is, Naranga aenescens, Mycalesis gotama, Marasmia patnalis, Marasmia exigua, Marasmia ruralis, Nymphula depunctalis, Scirpophaga innotata, Spodoptera litura, Chilo polychrysus, Rupela albinella, Diatraea saccharalis, Spodoptera frugiperda, Mythimna unipuncta, Chilo zacconius and Pamara guttata, most particularly Chilo suppressalis, Scirpophaga incertulas, Marasmia patnalis, Cnaphalocrocis medinalis, Agelastica alni, Hypera postica, Hypera brunneipennis, Haltica tombacina, Anthonomus grandis, Tenebrio molitor, Triboleum castaneum, Dicladispa armigera, Trichispa serica, Oulema oryzae, Colaspis brunnea, Lissorhorptrus oryzophilus, Phyllotreta cruciferae, Phyllotreta striolata, Psylliodes punctulata, Entomoscelis americana, Meligethes aeneus, Ceutorynchus sp., Psylliodes chrysocephala, Phyllotreta undulata, Leptinotarsa decemlineata, Diabrotica undecimpunctata undecimpunctata, Diabrotica undecimpunctata howardi, Diabrotica barberi, and Diabrotica virgifera.* Such cationic oligopeptides can also be delivered together with any dsRNA or siRNA molecules to insect cells *in vitro* or in cell culture (e.g., by adding them to the culture medium), or they may be mixed with dsRNA or siRNA molecules in the insect feed to be fed to insects, with the aim to silence certain insect target genes. Hence, these peptides are a useful research tool for the analysis of gene function, by mixing them (preferably in 1:1 ratio) with dsRNA or siRNA molecules targeting certain insect genes, e.g., in a process of pesticide target development as described above.

In this embodiment, the dsRNA or siRNA then targets an essential gene of this insect, preferably an essential gene that is expressed in the gut, and this dsRNA or siRNA and the cationic oligopeptide of the invention can be administered to an insect species cell culture *in vitro,* to a live insect, preferably to a larval stage thereof, by spraying in a field or by co-expression in a transgenic plant on which such insect species tends to feed.

In one embodiment of this invention, the current strategy is used in a plant which has already acquired partial or enhanced resistance to plant sap-sucking insects such as aphids or whiteflies, which plant is obtained by normal breeding and selection, or in a hybrid plant having better overall growth and vigour.

The dsRNAs or siRNAs of the current invention, and the method of applying them to the feed of insects such as plant sap-sucking insects, also have applications in insecticide development. New insecticides can be developed using the process of identifying and validating biological targets against which potential ligands can be screened (e.g., Margolis and Duyk, 1998). Production of new insecticides can be done using target-based discovery approaches. Genes that, when partially or entirely inactivated, kill the organism or significantly affect normal development when knocked out or repressed represent interesting targets. To identify compounds that have the same effect on the organism, high-throughput screening assays can be established to test compounds for their ability to interfere *in vitro* with the normal activity of the target. As such, the invention provides a method of identification of novel targets for insecticidal compounds, comprising the steps of: a) applying naked, unpackaged dsRNA or siRNA molecules to the feed or diet of a plant-sap sucking insect; b) analyzing which genes when silenced confer lethality to said insect, c) cloning and characterizing said genes thus analyzed; and optionally: d) identifying compounds that disrupt or inactivate said gene or the protein encoded thereby; and e) contacting said chemicals with said insect or feed or diet of said insect to confirm the pesticidal nature of said compound. In one embodiment of this invention, this unpackaged dsRNA or siRNA can be supplied together with a cationic oligopeptide as described herein for applications to any insect species.

Preferred target pests are plant sap-sucking insects, particularly of the order Hemiptera, preferably insects of the suborder Homoptera or Heteroptera, preferably aphids and whiteflies, particularly insects of the family of Aphididae. The invention is similarly applicable to any plant sap-sucking insects feeding from xylem by using a constitutive promoter or a xylem-specific promoter. Examples of xylem-specific promoters (i.e., promoter preferentially but not necessarily exclusively active in xylem) include but are not limited to: the Pal2 promoter, e.g. from bean (Leyva et al., 1992), the poxN or poxA promoters from rice (Ito et al., 2000), the PtCesA promoter (Wu et al., 2000), the XCP1 promoter (Funk et al., 2002), the cotton curl leaf virus promoter (Yingqiu et al., 2000), the vs-1 promoter element (Torres-Schumann et al., 1996).

"Plant sap-sucking insects" as used herein are insects feeding on plants using their sharp mouth parts which can be inserted into a plant to take fluid from the plant vascular system, in one embodiment these are insects feeding directly on the fluids in the plant vascular system, preferably insects only feeding on the fluids in the plant vascular system. In the insertion step, plant cells can also be damaged which may or may not be used as a food source by the plant sap-sucking insect. These insects are plant pests because their feeding reduces the vitality of the crops they feed on and they can transmit viral diseases. Also, many such sap-sucking insects secrete a sugar-rich fluid named honeydew that accumulates on lower plant parts, and such parts soon become covered by certain black or brown fungi known as sooty molds, hence interfering with plant photosynthesis.

Included in such plant sap-sucking insects are aphids or Homopteran insects of the Aphididae, and plant sap-sucking insects as used herein include but are not limited to the peach-potato aphid *Myzus persicae,* the bean aphid *Aphis fabae*, the pea aphid *Acyrthosiphum pisum*, the cabbage aphid *Brevicoryne brassicae,* the grain aphid *Sitobion avenae,* the rose-grain aphid *Metopolophium dirhodum,* the Russian wheat aphid *Diuraphis noxia* (Mordvilko), the English grain aphid *Macrosiphum avenae,* the greenbug aphid *Schizaphis graminum* (Rondani), the carrot aphid Cavariella *aegopodii,* the potato aphid *Macrosiphum euphorbiae,* the groundnut aphid *Aphis craccivora,* the cotton aphid *Aphis gossypii,* the black citrus aphid *Toxoptera aurantii,* the brown citrus apid *Toxoptera ciidius,* the willow aphid *Cavariella* spp., the corn leaf aphid *Rhopalosiphum maidis, the* aphid *Rhopalosiphum padi,* the willow leaf aphids *Chaitophorus* spp., the black pine aphids Cinara spp., the Sycamore Aphid *Drepanosiphum platanoides,* the Spruce aphids *Elatobium* spp., *Aphis citricola, Lipaphis pserudobrassicae* (turnip aphid), *Nippolachnus piri,* the foxglove aphid *Aulacorthum solani,* the asparagys aphid *Brachycorynella asparagi,* the brown ambrosia aphid *Uroleucon ambrosiae,* the buckthorn aphid *Aphis nasturtii,* the corn root aphid *Aphis maidiradicis,* the cresentmarked lily aphid *Neomyzus circumflexes,* the goldenglow aphid *Dactynotus rudbeckiae,* the honeysuckle and parsnip aphid *Hyadaphis foeniculi,* the leafcurl plum aphid *Brachycaudus helichrysi,* the lettuce root aphid *Pemphigus bursarius, the* mint aphid *Ovatus crataegarius,* the artichoke aphid *Capitophorus elaeagni,* the onion aphid *Neotoxoptera* formosana, the pea aphid *Macrosiphum pisi,* the rusty plum aphid *Hysteroneura* setariae, the shallot aphid *Myzus* ascalonicus, the solanum root aphid *Smynthurodes betae,* the sugarbeet root aphid *Pemphigus betae,* the tulip bulb aphid *Dysaphis tulipae,* the western aster root aphid *Aphis armoraciae,* the white aster root aphid *Prociphilus erigeronensis.*

Also included herein as plant sap-sucking insects are whiteflies or Aleyrodidae insects such as Trialeurodes vaporariorum (greenhouse whitefly), the banded wing whitefly Trialeurodes abutilonea, Bemisia tabaci (sweetpotato whitefly) and Bemisia argentifolli (silverleaf whitefly). Also included herein as plant sap-sucking insects are fleahoppers such as Pseudatomoscelis seriatus or cotton fleahopper, and Halticus bractatus or garden fleahopper, Pentatomidae (stink bugs, e.g., Thyanta spp.), mealybugs (Hemiptera, Coccoidea, Pseudococcidae, e.g., the citrus mealy bug (genus Pseudococcus)), as well as Delphacidae (or planthoppers) such as Laodelphax striatellus (small brown planthopper), Nilaparvata lugens (rice brown plant hopper) and Sogatella furcifera (white-backed rice planthopper), and Deltocephalidae (or leafhoppers) such as Flexamia DeLong spp., Nephotettix cincticeps and Nephotettix virescens, Amrasca bigutulla, and the potato leafhopper Empoasca filament. Also included are scales (also named scale insects) such as Aonidiella aurantii (California red scale), Comstockaspis pemiciosa (San Jose scale), Unaspis citri (citrus snow scale), Pseudaulacaspis pentagona (white peach scale), Saissetia oleae (brown olive scale), Lepidosaphes beckii (purple scale), Ceroplastes rubens (red wax scale) and Icerya purchasi (cottonycushion scale), besides Tingidae (or lace bugs) and Psyllidae insects, and spittle bugs.

Further included as plant sap-sucking insects are Heteropteran insects and Hemipteran insects of the Auchenorrhyncha that feed from the plants' vascular system, such as sap-sucking insects of the Cicadoidea (such as Cicadas), Cercopoidea (spittlebugs or froghoppers), Membracoidea (leafhoppers and treehoppers), and Fulgoroidea (planthoppers), e.g., the cotton seed sucker bug *Dysdercus peruvianus* (Heteroptera, Pyrrhocoridae), the apple dimpling bug, Campylomma liebknechti (Hemiptera:Miridae) and the green mirid, *Creontiades dilutus* which are cotton sucking insect pests, and the Lygus bugs (Hemiptera:Miridae, e.g., Lygus hesperus).

In one embodiment of this invention, phloem-feeding plant sap-sucking insects are targeted with the methods of the invention, particularly aphids, planthoppers and whiteflies.

The dsRNA or siRNA chimeric gene construct can also be present in a plant already expressing an insecticidal protein, e.g., an insecticidal protein derived from Bacillus thuringiensis. Preferred plants expressing such proteins include but are not limited to: corn plants containing the MON863 transformation event, corn plants containing the MON810 transformation event, corn plants containing the Bt11 transformation event, corn plants expressing a Cry1F protein, cotton plants expressing a Cry1Ac protein or cotton plants expressin a Cry1Ac and a Cry2Ab protein (Bollgard™ I or II), cotton plants expressing a Cry1 F, Cry1 F/1Ac hybrid protein or a VIP3A protein, and corn or cotton plants combining such transgenic events in the same plant species.

Preferred embodiments of the invention are shown in the below Examples which are a selection of possible embodiments and an illustration of the invention. It is evident that a variety of noncritical parameters can be changed to give the same or essentially the same result.

The following sequences are enclosed to this application in the sequence listing:
SEQ ID NO: 1 - designed degenerate primer DNA sequence of elF1A-F primer
SEQ ID NO: 2 - designed degenerate primer DNA sequence of elF1A-R primer (n at position 20 is uncertain and can be a, c, g, or t)
SEQ ID NO: 3 - designed degenerate primer DNA sequence of alpha-tubulin-F primer
SEQ ID NO: 4 - designed degenerate primer DNA sequence of alpha-tubulin-R primer
SEQ ID NO: 5 - DNA sequence of the *A. gossypii* elF1A gene PCR fragment
SEQ ID NO: 6 - DNA sequence of the *M. persicae* elF1A gene PCR fragment
SEQ ID NO: 7 - DNA sequence of the *A. gossypii* alpha-tubulin gene PCR fragment (n at positions 591, 592 and 637 is uncertain and can be a, c, g, or t)
SEQ ID NO: 8 - DNA sequence of the *M. persicae* alpha-tubulin gene PCR fragment (n at positions 3, 113, 128, 137, 509, 615, 617, and 627 is uncertain and can be a, c, g, or t)
SEQ ID NO: 9 - designed primer sequence of the *Myzus persicae* EcR C domain forward primer
SEQ ID NO: 10 - designed primer sequence of the *Myzus persicae* EcR E domain reverse primer
SEQ ID NO: 11 - DNA sequence of the *Myzus persicae* EcR PCR fragment
SEQ ID NO:12 - DNA sequence of the Myzus persicae alpha-actinin gene fragment (n at position 704 is uncertain and can be a, c, g or t).

Unless stated otherwise in the Examples, all recombinant DNA techniques are carried out according to standard protocols as described in Sambrook and Russell (2001) Molecular Cloning: A Laboratory Manual, Third Edition, Cold Spring Harbor Laboratory Press, NY, in Volumes 1 and 2 of Ausubel et al. (1994) Current Protocols in Molecular Biology, Current Protocols, USA and in Volumes I and II of Brown (1998) Molecular Biology LabFax, Second Edition, Academic Press (UK). Standard materials and methods for plant molecular work are described in Plant Molecular Biology Labfax (1993) by R.D.D. Croy, jointly published by BIOS Scientific Publications Ltd (UK) and Blackwell Scientific Publications, UK. Standard materials and methods for polymerase chain reactions can be found in Dieffenbach and Dveksler (1995) PCR Primer. A Laboratory Manual, Cold Spring Harbor Laboratory Press, and in McPherson at al. (2000) PCR - Basics: From Background to Bench, First Edition, Springer Verlag, Germany. Detailed information and references on RNA interference can be found on the website of Ambion, at: www.ambion.com.

### EXAMPLES

### Example 1: injection and feeding of aphids with dsRNA

### Insect rearing

*Aphis gossypii* were raised on 3-5 week old cotton plants, at 25°C under a 14 hour:10 hour day/night cycle, with low to medium humidity. Between 5 and 10 apterae adults were placed on each plant on a weekly basis. *Myzus persicae* were raised on 3-5 week old radish plants, at 20°C under a 14 hour:10 hour day/night cycle with low to medium humidity. Between 10 and 15 apterae adults were placed on each plant every two weeks.

### PCR amplification and sequence analysis of aphid genes

Total RNA was extracted from a population of aphids of mixed life stages using a Qiagen RNeasy Minikit™, according to the manufacturer's specifications. The RNA (-250 ng) was used to prepare cDNA using Invitrogen's Thermoscript RT-PCR System™, using oligo(dT) primers. The resulting cDNA was then used as template for the amplification of the eukaryotic initiation factor.

Degenerate PCR primers were designed to span moderate to highly conserved regions of the eukaryotic initiation factor 1A (elF 1A) gene and the alpha-tubulin genes, based on alignments of multiple sequences of invertebrate's genes derived from GenBank. Sequences were aligned and multiple sequence comparisons were generated using either the GCG program 'Pileup' or 'CLUSTAL W' with default parameters for the nucleotide sequences and the default-scoring matrix for proteins. Primers were designed to cover a single putative exon whenever possible. Exon predictions were usually based on exon boundaries found in the *Drosophila melanogaster* orthologue of the target gene. To assist with the design process, the CODEHOP program (Blocks Server, http://www.blocks.fhcrc.org) was used.

**Table 1. PCR primers used to isolate gene sequences from A. gossypii and M. persicae**

| Primers | Primer sequence |
|---|---|
| elF 1A primers: | |
| EIF1A-F (SEQ ID NO:1) | 5' AAA ACA GAA GAA GAG GTA AAA AYG ARA 3' |
| EIF1A-R (SEQ ID NO:2) | 5' GGT TTC TGG CTT CGT CTG GNG TRT AYT T 3' |
| Alpha-tubulin primers: | |
| Tubulin-F (SEQ ID NO:3) | 5' TAC AAC TCS ATC YTG ACC AC 3' |
| Tubulin-R (SEQ ID NO:4) | 5' TCC ATR CCY TCW CCB ACR TAC C 3' |

Amplifications of the target genes were performed using Invitrogen's Thermoscript RT-PCR System, with Platinum *Taq* DNA polymerase, on a Corbett Research Thermocycler. A range of annealing temperatures (0.5 to 5 degrees below the predicted Tₘ of the primers), and a range of MgCl₂ concentrations (0 to 3.0 mM) were tested to find the conditions that produced the maximum amount of specific PCR product. The following PCR conditions were used: 94°C for 2 min, 30 cycles of [94°C for 30 sec; 47-52°C for 30 sec; 72°C for 90 sec], and 72°C for 3 min.

The PCR products were gel purified using a Perfectprep Gel Cleanup Kit (Eppendorf) and subcloned into the plasmid pGEM-T-Easy (Promega). Isolated plasmid DNA was sequenced using primers complementary to the T7 and SP6 promoters in the vector. DNA was sequenced using BigDye-terminator chemistry (Applied BioSystems) according to the manufacturer's specifications, and sequencing reactions were resolved and processed by an Applied BioSystems Model377XL automated DNA sequencer.

DNA sequences were edited to remove plasmid sequences and pairwise comparisons were performed using the GCG alignment program 'Gap' (Devereux et al., 1984) and multiple sequence comparisons and consensus sequences were generated using either the GCG program 'Pileup' (Devereux et al., 1984) or 'CLUSTALW' (Thompson et al., 1994) with the default parameters (gap weight 5.0, gap length weight 0.3) for nucleotide sequences.

A single 279 nt long PCR product was amplified from *A*. *gossypii* and *M. persicae* cDNA using the aforementioned elF primers. Each PCR product was sequenced, and based on DNA sequence comparisons with other known invertebrate elF genes, both were confirmed to be elF gene fragments. SEQ ID NO: 5 and 6 show the DNA sequences of the *A. gossypii* and *M. persicae* elF 1A PCR fragments, respectively.

The sequences of the alpha-tubulin *A. gossypii* and *M*. *persicae* PCR fragments corresponding to the genes found in the cDNA library of these insects is shown in SEQ ID NO: 7 and 8, respectively.

Using the above protocol, also DNA sequences for the *A*. *gossypii* alpha-actinin, acetylcholinesterase and elongation factor 1-alpha gene are determined, based on the available sequences of the *Drosophila* orthologs of these gene in Genbank accession numbers NM058137 (alpha-actinin), X05893 (acetylcholinesterase), or based on the available sequence of the numbers M. Persicae ortholog of the elongation factor 1 alpha gene in Genbank accession number AF143612.

Using a similar procedure as described above for the elF and alpha-tubulin genes, also a DNA sequence of a *Myzus persicae* alpha-actinin gene was isolated. This sequence is shown in SEQ ID NO: 12.

### Preparation of double-stranded RNA

To facilitate in vitro dsRNA synthesis, the gene fragment from the pGEM-T-Easy plasmid was subcloned into the pL4440 plasmid (Timmons and Fire, 1998), a plasmid with two convergent T7 promoters. Transcription from this plasmid requires the use of only T7 RNA polymerase to produce both sense and antisense RNAs simultaneously, which will anneal in vitro to form dsRNA.

The plasmid pL4417 (described in Fire Lab 1997 Vector Supplement, February 1997, provided by Andrew Fire, Carnegie Institute; Timmons et al., 2001) contains the Aequorea victoria green fluorescent protein gene, gfp, flanked by two convergent T7 promoters. This plasmid was used to prepare dsRNA of the entire (716 nt) open reading frame of the gfp gene, to assess whether non-aphid specific dsRNA had any deleterious effects on the aphids.

To obtain sufficient DNA template for in vitro transcriptions, the aphid gene fragment and flanking T7 promoter sequences were PCR-amplified from the pL4440 plasmid using plasmid-specific primers.

Double-stranded RNA was prepared using a T7 RiboMAX Express Large Scale RNA production System (Promega) according to the manufacturer's specifications.

### Insect Bioassays

To test if the *A*. *gossypii* elF1A dsRNA was functional in silencing the aphid gene, haemocoel injections of dsRNA targeting this aphid gene were done in *A*. *gossypii* adult females and the mortality in time and the fecundity was compared to control, buffer-injected or dsGFP (green fluorescent protein)-injected *A*. *gossypii.* 25 apterae adults were injected with 1 microg/microl dsRNA in injection buffer (5mM KCI, 0.1 M sodium phosphate, pH 6.8). Ten apterae adults were similarly injected with dsGFP (1 microg/microl) in injection buffer and 10 apterae adults were injected with injection buffer only. Injected aphids were placed individually in arenas containing the artificial aphid diet described below. Mortality of the adults and the number of live nymphs were recorded at 3, 7, 11, and 14 days post injection, after which the experiment was terminated. This regime was repeated three times such that a total of 75 adults had been injected with the aphid-specific dsRNA. Injection of elF dsRNA resulted in a considerable percentage (29 %) of A. gossypii adults dying within 3 days following the injection, and the remaining injected aphids continued to die over the following 2 weeks. In comparison, considerable fewer aphids died at 3 days post injection when injected with either GFP dsRNA (3 %) or injection buffer (7 %).

Injections of *M*. *persicae* with *M*. *persicae* elF dsRNA also killed some aphids, but the mortality rates in this experiment did not appear to be significant when compared to GFP dsRNA- or buffer-injected controls (a large variation was observed in the controls and the treatment).

For those *A. gossypii* females that survived the injection treatment, their nymph production was assessed over a two week period. The cumulative number of nymphs produced at 7, 11, and 14 days post injection were similar for all treatments, with the aphid-specific dsRNA having no significant effect on the reproduction of the treated females that survived.

Similarly, a portion of the Myzus persicae ecdysone receptor (EcR) gene was amplified from M. persicae cDNA using the following PCR primer sequences:
MpEcR C domain forward primer: 5' CCCAAGCTTTGCCTGGTGTGTGGCGACCGG 3' (SEQ ID NO:9).
MpEcR E domain reverse primer: 5' CCCAAGCTTATCCTGGAAATAGACAAGTCG 3' (SEQ ID NO:10).

The underlined adapters were added to provide a HindIII site for subcloning into a dsRNA expression vector.

The about 450 bp PCR product was gel-purified using a Perfectprep Gel Cleanup Kit (Eppendorf) and subcloned into the plasmid pGEM-T-Easy (Promega). The gene fragments from the pGEM-T-Easy plasmid were subcloned into the pL4440 plasmid (kindly provided by Andrew Fire), a plasmid with two convergent T7 promoters. Transcription from this plasmid requires the use of only T7 RNA polymerase to produce both sense and antisense RNAs simultaneously, which will anneal in vitro to form dsRNA. Double-stranded RNA was prepared using a T7 RiboMAX Express Large Scale RNA production System (Promega) according to the manufacturer's specifications.

SEQ ID NO:11 shows the Myzus persicae ecdysone receptor DNA sequence coding from the start of the C domain to the 15th residue of the E domain (about 450 bp).

Two to three day old adult females were microinjected into their abdominal haemocoels with 50 nL of buffer, with or without 1 ug/uL dsRNA (elF1A or EcR). Aphids were anaesthetised using CO2, and secured to a microscope slide coated with a dried, sticky layer of 20% sucrose. The insects were injected using borosilicate glass needles, prepared using a micropipette puller (Sutter Instruments) and sometimes sharpened with a needle beveller (Narashige). The needle was operated using a micromanipulator (Narashige) secured to a stereomicroscope, and the fluid was delivered with the aid of an air-driven pump controlled by a foot-operated solenoid switch. Injected aphids were washed from the sucrose pad, and transferred to individual feeding arenas. The arenas consisted of small (1.5 cm inner diameter) plastic cylinders closed at one end with a glass microscope coverslip and at the other end with two Parafilm membranes. An artificial liquid diet (see below) was contained between the two Parafilm membranes, and the survival of the injected aphids, and the number of nymphs that they produced over a 10-day period was monitored.

No increase in aphid mortality was observed for M. persicae aphids injected with EcR dsRNA. Aphids injected with buffer only or with elF dsRNA produced a similar number of nymphs at days 5 and 10 post injection (Figure 1). Aphids injected with EcR dsRNA produced significantly fewer nymphs after day 10 (n=25, Student's T-test, P<0.01), suggesting that although the injected dsRNA had taken some time to promote an effect on nymph production, it adversely affected insect fecundity.

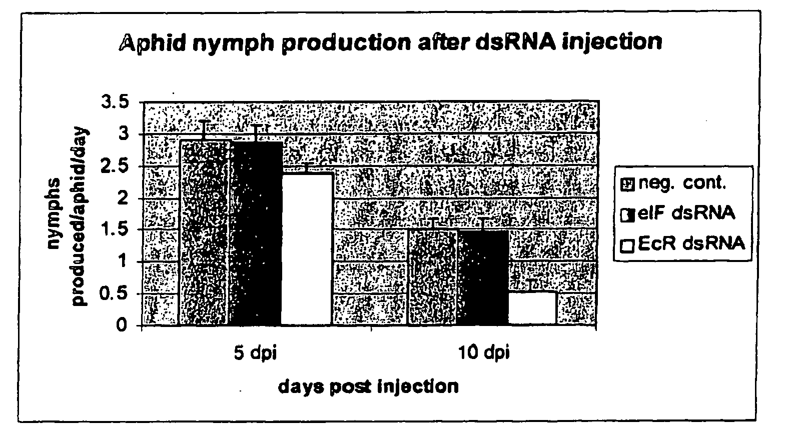

Figure 1. Nymph production of adult female M. persicae injected with dsRNA. Aphids were injected with buffer alone (neg cont.) or with elF or EcR dsRNA. The results represent the means and standard errors for n = 25 aphids for each of the three treatments.

Also, injection of Aphis gossypii alpha-tubulin dsRNA in the haemocoel of these aphids shows that aphid mortality is increased compared to control dsGFP injections. Hence, the *A*. *gossypii* tubulin sequence of SEQ ID NO: 7 or the gene corresponding to this sequence is another possible target gene.

For the feeding assays, three to four adult female *A*. *gossypii* aphids were placed in individual feeding arenas and left for 24 h. The adults were then removed, leaving the newborn nymphs to develop entirely on the artificial diets containing dsRNA. The arenas consisted of small (1.5 cm inner diameter) plastic cylinders closed at one end with a glass microscope coverslip or plastic film and at the other end with two Parafilm membranes. A total of 100 microliter of artificial liquid diet was contained between the two Parafilm membranes, containing either 1 microgram/microliter dsRNA (gfp or elF) or 10 microliter RNA dilution buffer (10 mM Tris, pH 8.0). Three to five independent preparations of elF and gfp dsRNA were pooled to provide sufficient quantities of dsRNA (5 mg) for the feeding bioassays. The artificial diet was prepared as a 2x stock, so that following addition of dsRNA and buffer, the final concentration of diet could be diluted to 1x concentration by adding the appropriate volume of water. Between 45 and 50 arenas were set up for each treatment (buffer, gfp-dsRNA, and elF-dsRNA), starting with, on average, 40 nymphs per arena at day 1. The number of live aphids at days 3, 7, and 11 were recorded, and the percentage of aphids surviving for each arena, relative to day 1, was calculated.

The artificial diet used includes, for 9ml (all ingredients from cell culture quality): amino acid stock solution (1ml), vitamin stock solution (0.3 ml), sucrose 80% (3.125 ml), potassium-sodium phosphate buffer (1 ml), ovalbumin (10 mg), MgCl₂ (10mg), Wesson salts (10mg), water (3.57 ml). The potassium-sodium buffer solution (100mM, pH 7.0) contains: NaH₂PO₄: 1.4 g, K₂HPO₄: 2.6 g, Ascorbic Acid: 0.25 g, adjust pH to 7.0, mix with 250ml water (this is not stored, an amount is made to use immediately in the diet which is stored). The diet is mixed, filtered on 0.2µm filters and stored in 5ml tubes at -70°C. A large amount is prepared, which is stored in the freezer for ±6 months. Just before a bioassay, antibiotics are added: to 9ml of diet, 20µl erythromycine (10mg/ml EtOH), 10µl triacilline (100mg/ml), 5µl chloramphenicol (60mg/ml), 6µl kanamycine (50mg/ml). This has some negative effect on growth of larvae and can be omitted when working with cell-free samples.

The amino acid stock solution contains (all L-amino acids, between brackets final concentration in mM are indicated): Glutamine (32.8), Serine (25.5), Arginine (10.7), Valine (7.1), Threonine (6.9), Leucine (5.1), Lysine (5.1), Isoleucine (4.4), Phenylalanine (2.8), Histidine (2.2), Methionine (1.6), Alanine (1.6), Tryptophan (0.9).

The vitamin stock solution contains (between brackets mg/10 ml): biotin (0.1), Capathotenate (5), choline chloride (50), myo-inositol (50), niacin (10), pyridoxin (2.5), thiamine-HCl (2.5).

The amino acid stock solution and the vitamin stock solution are not stored: an amount is prepared to use immediately in the diet which is stored.

Feeding elF1A dsRNA to *A*. *gossypii* nymphs resulted in a significant reduction in the survival of nymphs (Figure 2). After 11 days, only 17 (± 1.4) % of the aphids fed on diet containing elF dsRNA had survived, whereas 54 (± 4.1) and 51 (± 2.8) % of the aphids fed on diet containing buffer or gfp dsRNA had survived. Hence, there is a two-fold increase in aphid mortality due to the presence of the elF dsRNA in the diet. Student t-tests confirmed that the feeding of elF dsRNA significantly reduced the survivorship of aphids, relative to aphids fed either buffer or gfp dsRNA (P< 0.0001).

Figure 2. Survival of *A*. *gossypii* on diet containing dsRNA. Neonate aphids (approximately 40/arena) were fed diet containing 1.0 microgram/microliter dsRNA (gfp or elF), and the number of surviving aphids was assessed at days 3, 7 and 11. The percentage of aphids surviving (relative to day 1) was calculated. The values represent the mean +/- standard error for >45 replicates).

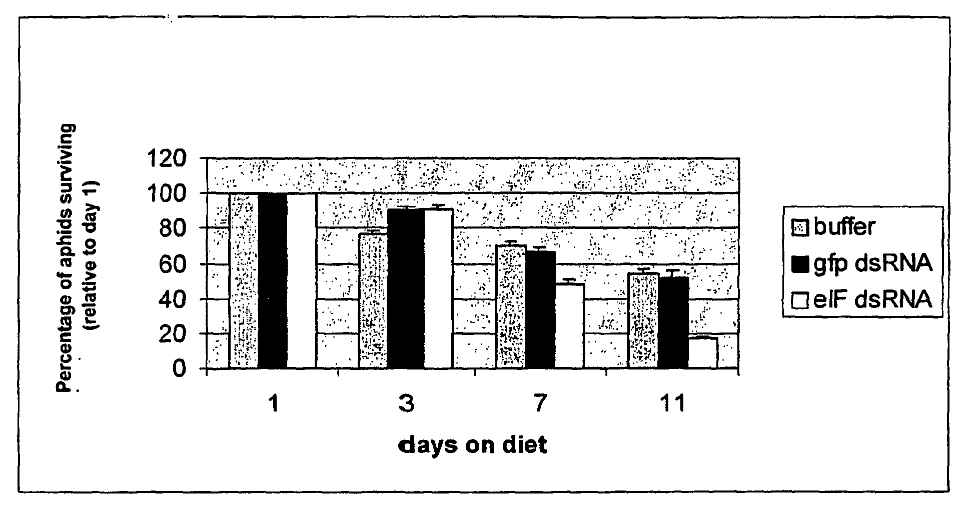

These data of a significantly higher mortality of aphids when fed dsRNA have been confirmed in another experiment wherein a smaller number of aphids was used, but using a similar experimental setup as above (also using the *A. gossypii* elF1A dsRNA fed to *A*. *gossypii* nymphs).

A similar experimental setup with *M. persicae* aphids and a dsRNA based on the above M. persicae elF1A sequence (SEQ ID NO:6) confirms that a significant mortality is obtained when feeding these aphids naked, unpackaged dsRNA molecules in their diet.

These results show that surprisingly, significant mortality is found in aphids fed naked, unpackaged dsRNA targeted to an essential aphid gene.

Also, insect mortality and efficient silencing is obtained by using small interfering RNA molecules (siRNA) directly in the aphid diet. For 3 target genes (elF, tubulin and GFP), siRNA is prepared by long dsRNA cleavage using the RNAselli (Ambion's Silencer siRNA Cocktail Kit, generating 12-30 bp siRNAs) or recombinant human dicer (Gene Therapy Systems, producing (more optimal) 21-23 bp siRNAs). Aphids are fed artificial diet containing a concentration of long dsRNA and siRNAs to determine the difference in efficacy. Application of siRNA molecules in the aphid diet also produces a significant effect on aphid development, comparable to or even better then the longer dsRNA applied above.

Addition of a poly-Arginine 12-mer cationic oligopeptide to the aphid diet, together with the elF1A dsRNA, results in a significantly increased mortality in the above feeding assays towards *A*. *gossypii* and *M*. *persicae,* compared to the application of dsRNA without such peptide and a control setup using GFP dsRNA.

### Example 2: analysis of aphid feeding on plants transiently expressing dsRNA.

To confirm that plants can deliver dsRNA or siRNA to plant sap-sucking insects such as aphids *in planta* without transfection promoting agents, aphids are added to tobacco plants transiently induced to produce aphid dsRNA using the SVISS methodology described in published PCT application WO 03/052108, using dsRNA targeted to a *M. persicae* essential gene.

These experiments confirm that the dsRNA transiently produced in plants in an unpackaged form, free from transfection-promoting agents, can result in a significant inhibition of *M*. *persicae* development.

### Example 3: analysis of aphid feeding on plants stably expressing dsRNA in phloem.

In this comparative experiment, *Arabidopsis* and tobacco plants are transformed by *Agrobacterium-*mediated transformation with a vector containing a dsRNA chimeric gene targeting either the *M. persicae* elF1A gene, the *M. persicae* ecdyson receptor (EcR) or the control GFP gene. A *M. persicae* ecdyson receptor DNA sequence was cloned from this aphid as described above. A plant dsRNA chimeric gene is made wherein either the phloem-specific rolC promoter (Pandolfini et al., 2003) or the constitutive CaMV 35S promoter is operably linked to the dsRNA construct, containing sense and antisense regions to the above target aphid gene or the control gene. Plants are tested for transformation using Southern blot analysis and for expression and dicing of the dsRNA by small RNA Northern blot assay. Transport of interfering RNAs to the phloem is confirmed by the significant reduction in development of *Myzus persicae* feeding on the successfully transformed *Arabidopsis* and tobacco plants. Northern blot analysis confirms the presence of the RNA molecules of the invention in the phloem sap of plants that are successfully transformed.

Hence, plant sap-sucking insects can now be controlled using specific and selective dsRNA-sequences targeting essential plant sap-sucking genes so as to minimize population build-up of aphids on crop plants.

### Cited references

Adams and Sekelsky (2002) Nature Reviews Genetics 3:189.
Almon et al.(1997) Plant Physiol. 115:1599-1607.
An et al. (1996) The Plant J. 10, 107.
Ashford et al. (2000) J. Ins. Physiol. 46:335-341.
Bender et al. (1997) Cell. 91:777-88.
Brears et al. (1991) Plant J. 1:235-244.
Ceci et al. (2003) The Plant J. 33:557-566.
Christensen et al. (1992) Plant Mol. Biol. 18, 675-689.
Christou et al. (1990) Trends Biotechnology 8, 145.
Clarkson and Saint (1999) DNA Cell. Biol. 18:457-62.
Clarkson et al. (1999) Nature 399:694-7.
Comejo et al. (1993) Plant Mol. Biol. 23, 567-581.
Cristofoletti et al. (2003) J. Ins. Physiol. 49:11-24.
Datta et al. (1990) Bio/Technology 8, 736-740
Dehio et al. (1993) Plant Mol. Biol. 23:1199-1210.
de Pater et al. (1992) The Plant J. 2, 834-844.
Depicker et al. (1982) J. Molec. Appl. Genetics 1, 561-573.
Devereux J, et al. (1984) Nucleic Acids Res. 12:387-395.
Down et al. (1996) J. Insect Physiol. 42:1035-1045.
Dubreuil and Wang (2000) J. Muscle Res. Cell Motil. 21:705-13.
Edwards et al.(1990) Proc. Natl. Acad. Sci. USA 87:3459-3463.
Foissac et al. (2002) Insect Biochem. Mol. Biol. 32:967-978.
Franck et al. (1980) Cell 21, 285-294
Fromm et al. (1990) Bio/Technology 8, 833-839
Funk et al. (2002) Plant Physiol. 128:84-94.
Fyrberg et al. (1998). Biochem. Genet. 36:299-310
Gardner et al. (1981) Nucleic Acids Research 9, 2871-2887
Gielen et al. (1984) EMBO J. 3:835-845
Gordon-Kamm et al. (1990) The Plant Cell 2, 603-618
Graham et al. (1997). Plant Mol. Biol. 33:729-735.
Guivarc'h et al. (1996). Transgenic research 5:3-11.
Gura (2003) Nature 404:804-808.
Hay (2000) Cell. Death Differ. 7:1045-56.
Hedley et al. (2000) J. Exp. Botany 51:817-821.
Hehn and Rohde (1998). J. Gen. Virol. 79:1495-1499.
Henrich et al. (2000) Genesis 28:125-33.
Hinchee et al. (1988) Bio/Technology 6, 915.
Hovemann et al. (1988) Nucl. Acids Res. 16:3175-94.
Hull and Howell (1987) Virology 86, 482-493.
Ito et al.(2000) Plant Science 155:85-100.
Järver and Langel (2004) Drug Discovery Today 9 :395-402.
Jiang et al. (2000). Dev. Biol. 226:118-36.
Johnson et al. (1998) Gene 221:127-34.
Kertbundit et al. (1991) Proc. Natl. Acad. Sci. USA 88: 5212-5216.
Kiyokawa et al. (1994) Plant Physiology 104:801-802.
Korber et al.(1991) EMBO J. 10:3983-91.
Kuhn et al. (1997) Science 275:1298-1300.
Lam and Thummel (2000) Curr. Biol. 10:957-63.
Last et al. (1990) Theor. Appl. Genet. 81, 581-588.
Leyva et al. (1992) Plant Cell. 4: 263-271.
Magrelli et al. (1994) Science 266:1986-1988.
Margolis and Duyk (1998) Nature Biotech. 16:311.
Marzoui et al. (1999) RNA 5:1615-31.
Matthews and Kaufman (1987) Dev. Biol. 119:100-114
Medberry et al. (1992) Plant Cell 4:185-192.
Moran and Thompson (2001) Plant Physiol. 125:1074-1085.
Mottus et al. (2000) Genetics 154:657-68.
Myrick and Dearolf (2000) Gene 244:119-125.
Needleman and Wunsch (1970) J. Mol. Biol. 48:443-53.
Newmark et al.(2003) P.N.A.S. USA 100, suppl. 1, 11861-11865.
Nolte and Koch (1993) Plant Physiol. 101:899-905.
Pandolfini et al. (2003) BioMedCentral (BMC) Biotechnology 3:7 (http://www.biomedcentral.com/1472-6750/3/7)
Pathirana et al. (1997). Plant J. 12:293-304.
Rahbé et al. (1995) Entomologia Experimentalis et Applicata 76:143-155.
Rajagopal et al. (2002). J. Biol. Chem. 277:46849-46851.
Ramloch-Lorenz et al. (1993). The Plant J. 4:545-554.
Rao et al. (1998) The Plant Journal 15:469-477.
Rohde et al. (1994) Plant Mol. Biol. 27:623-628.
Rossi et al. (1998) Proc. Natl. Acad. Sci. USA 95:9750-9754.
Roy et al. (2002) J. Agric. Food Chem. 50:6775-9.
Schunmann et al. (2003) Plant Functional Biology 30:453-460.
Shi et al.(1994) J. Exp. Bot. 45:623-631.
Shigenobu et al. (2000) Nature 407:81-86.
Shimamoto et al. (1989) Nature 338, 274-276.
Smith et al. (2000) Nature 407:319-20.
Spena and Langenkemper (1997) Genet. Res. 69:11-15.
Spradling et al. (1999) Genetics 153:135-177.
Stoger et al. (1999) Molecular Breeding 5:65-73.
Thompson et al. (1994) Nucleic Acids Res. 22:4673-4680.
Timmons et al. (2001) Gene 263, 103-112.
Timmons and Fire (1998) Nature 395:854.
Tornero et al.(1996) Plant J. 9:639-648.
Torres-Schumann et al. (1996) Plant J. 9:283-96.
Tortiglione et al. (2003) Plant Mol Biol. 53(6):891-902.
Unnamalai et al. (2004) FEBS Letters 566:307-310.
van Daal and Elgin (1992) Mol Biol Cell 3:593-602.
Velten and Schell (1985) Nucleic Acids Research 13, 6981-6998 (1985)
Velten et al. (1984) EMBO J 3, 2723-2730 (1984).
Verdaguer et al. (1998) Plant Mol. Biol. 37, 1055-1067 (1998).
Wu et al. (2000) Plant J. 22:495-502.
Yin and Beachy (1995) Plant J. 7:969-980.
Yin et al. (1997) Plant J. 12:1179-88.
Yingqiu et al. (2000) Science in China (Series C) vol. 43:498-506.
Yoshimoto et al. (2003) Plant Physiol. 131:1511-1517.
Zeremski et al. (2003) Genesis 35:31-38.
Zhang et al. (1991) The Plant Cell 3, 1155-1165.
Zhang et al. (1996) Plant Physiol. 112:1111-1117.
Zhou et al. (1998) Chin. J. Biotechnol. 14:9-16.

## Claims

1. A method to silence a gene of a plant sap-sucking insect, comprising applying to the diet or feed of said plant sap-sucking insect dsRNA or siRNA without transfection-promoting agents, which dsRNA or siRNA is targeted to an essential plant sap-sucking insect gene.

2. A method of identifying gene function in a plant sap-sucking insect, comprising the step of applying dsRNA or siRNA targeting a plant sap-sucking insect gene to the diet of said insect, and evaluating phenotypic or biochemical changes in said insect, wherein said dsRNA or siRNA is applied without transfection-promoting agents.

3. A method of identification of novel targets for insecticidal compounds, comprising the steps of: a) applying dsRNA or siRNA without transfection-promoting agents to the feed or diet of a plant-sap sucking insect; b) analyzing which genes when silenced confer lethality to said insect, c) cloning and characterizing said genes thus analyzed; d) identifying compounds that disrupt or inactivate said gene or the RNA or protein encoded thereby; and e) contacting said compounds with said insect or feed or diet of said insect to confirm the pesticidal nature of said compound.

4. A method to obtain significant insect mortality or significant insect control by silencing of an essential gene of a plant sap-sucking insect by application of dsRNA or siRNA to the feed of said insect, as compared to control insects fed on dsRNA or siRNA targeting a non-essential gene or a gene not expressed in the plant sap-sucking insect.

5. The method of any one of claims 1, 2 or 4, wherein said sap-sucking insect gene is homologous to a gene which when partially or completely silenced in an insect such as *Drosophila* results in a mutant with a lethal phenotype, and wherein 2 genes are homologous when they are similar in sequence to such a degree that when the two sequences are aligned, the number of positions with identical nucleotides divided by the number of nucleotides in the shorter of the sequences is higher than 70%.

6. The method of any one of claims 1, 2 or 4, wherein said sap-sucking insect gene is a gene the silencing of which brings about a decreased growth, development, reproduction or survival of a plant sap-sucking insect compared to control insects fed on dsRNA or siRNA targeting a gene not expressed in the plant sap-sucking insect.

7. The method of any one of claims 1, 2, 4 to 6, wherein said dsRNA or siRNA does not silence genes of a plant, or of non-target animals, such as plant sap-sucking insect predators or animals such as reptiles, amphibians, birds, or mammals.

8. The method of any one of claims 1, 2, 4 to 7 wherein a portion of a target sequence is selected which is present in several plant sap-sucking insects of a plant host in identical sequence or with high sequence identity, wherein a sequence with high sequence identity refers to the number of positions with identical nucleotides divided by the number of nucleotides in the shorter of the sequences, being higher than 95 %.

9. The method of any one of claims 1, 2, 4 to 8, wherein said sap-sucking insect gene is selected from the group consisting of: the eukaryotic translation initiation factor 1A (elF1A), an (alpha) tubulin gene, an alpha-actin gene, and an ecdysone receptor gene.

10. The method of any one of claims 1, 2, 4 to 9, wherein said plant sap-sucking insect gene are those plant sap-sucking insect genes identified using the primers of any one of SEQ ID NO:1-4 and SEQ ID NO:9 and 10, or plant sap-sucking insect genes corresponding to or comprising any one of the sequences of SEQ ID NO:5 to 8, SEQ ID NO:11 or SEQ ID NO:12, as well as aphid genes homologous to the sequences of SEQ ID NO:5 to 8, SEQ ID NO:11 or SEQ ID NO:12, wherein 2 genes are homologous when they are similar in sequence to such a degree that when the two sequences are aligned, the number of positions with identical nucleotides divided by the number of nucleotides in the shorter of the sequences is higher than 70%.

11. The method of claim 10, wherein the dsRNA or siRNA targets the A. gossypii elF1A sequence of SEQ ID NO:5 only in the portion from nucleotide position 72 to the end in SEQ ID NO:5.

12. The method of any one of claims 1, 2, 4 to 9, wherein said plant sap-sucking insect gene comprises the sequence of any one of SEQ ID NO:5 to 8, or SEQ ID NO:12.

## Patentansprüche

1. Verfahren für das Silencing eines Gens eines pflanzensaftsaugenden Insekts, bei dem man dsRNA oder siRNA ohne transfektionsfördernde Mittel auf die Nahrung oder das Futter dieses pflanzensaftsaugenden Insekts aufbringt, wobei mit dsRNA oder siRNA ein Targeting an ein essentielles Gen eines pflanzensaftsaugenden Insekts erfolgt.

2. Verfahren zum Identifizieren einer Genfunktion in einem pflanzensaftsaugenden Insekt, umfassend den Schritt, dass man dsRNA oder siRNA, mit der ein Targeting eines Gens eines pflanzensaftsaugenden Insekts erfolgt, auf die Nahrung des Insekts aufbringt und phänotypische oder biochemische Veränderungen bei dem Insekt auswertet, wobei die dsRNA oder siRNA ohne transfektionsfördernde Mittel aufgebracht wird.

3. Verfahren zum Identifizieren von neuen Targets für insektizide Verbindungen, umfassend die folgenden Schritte: a) Aufbringen von dsRNA oder siRNA ohne transfektionsfördernde Mittel auf das Futter oder die Nahrung eines pflanzensaftsaugenden Insekts; b) Analysieren, welche Gene für das Insekt letal sind, wenn bei ihnen ein Silencing erfolgt; c) Klonieren und Charakterisieren der so analysierten Gene; d) Identifizieren von Verbindungen, die das Gen oder die/das davon kodierte RNA oder Protein disrumpieren oder inaktivieren; und e) Inkontaktbringen der Verbindungen mit dem Insekt oder dem Futter oder der Nahrung des Insekts, um die Pestizidwirkung der Verbindung zu bestätigen.

4. Verfahren zum Erhalt einer nennenswerten Mortalität eines Insekts oder einer nennenswerten Bekämpfung eines Insekts durch Silencing eines essentiellen Gens eines pflanzensaftsaugenden Insekts durch Aufbringen von dsRNA oder siRNA auf das Futter des Insekts, im Vergleich zu Kontrollinsekten, denen dsRNA oder siRNA, mit der Targeting eines nichtessentiellen Gens oder eines Gens, das in dem pflanzensaftsaugenden Insekt nicht exprimiert wird, erfolgt, als Futter gereicht wird.

5. Verfahren nach einem der Ansprüche 1, 2 oder 4, wobei das Gen eines pflanzensaftsaugenden Insekts zu einem Gen, das, wenn mit ihm in einem Insekt, wie zum Beispiel bei *Drosophila,* ein teilweises oder vollständiges Silencing erfolgt, zu einer Mutante mit einem letalen Phänotyp führt, homolog ist, und wobei zwei Gene homolog sind, wenn sie in einer Sequenz so stark ähnlich sind, dass, wenn mit den beiden Sequenzen ein Alignment erfolgt, die Anzahl Positionen mit identischen Nukleotiden dividiert durch die Anzahl Nukleotide in der kürzeren der Sequenzen mehr als 70% beträgt.

6. Verfahren nach einem der Ansprüche 1, 2 oder 4, wobei es sich bei dem Gen eines pflanzensaftsaugenden Insekts um ein Gen handelt, dessen Silencing zu einem verringerten Wachstum, einer verringerten Entwicklung, einer verringerten Reproduktion oder einem verringerten Überleben eines pflanzensaftsaugenden Insekts verglichen mit Kontrollinsekten, denen dsRNA oder siRNA, mit der ein Targeting eines Gens, das in dem pflanzensaftsaugenden Insekt nicht exprimiert wird, erfolgt, als Futter gereicht wird, führt.

7. Verfahren nach einem der Ansprüche 1, 2, 4 bis 6, wobei mit der dsRNA oder siRNA kein Silencing von Genen einer Pflanze oder von Nontarget-Tieren, wie Prädatoren eines pflanzensaftsaugenden Insekts, oder Tieren wie Reptilien, Amphibien, Vögeln oder Säugetieren, erfolgt.

8. Verfahren nach einem der Ansprüche 1, 2, 4 bis 7, wobei ein Abschnitt einer Target-Sequenz gewählt wird, die in mehreren pflanzensaftsaugenden Insekten einer Wirtspflanze in einer identischen Sequenz oder mit hoher Sequenzidentität vorhanden ist, wobei eine Sequenz mit hoher Sequenzidentität bedeutet, dass die Anzahl Positionen mit identischen Nukleotiden dividiert durch die Anzahl Nukleotide in der kürzeren der Sequenzen mehr als 95% beträgt.

9. Verfahren nach einem der Ansprüche 1, 2, 4 bis 8, wobei das Gen des saftsaugenden Insekts aus der Gruppe bestehend aus: dem Eukaryonten-Translationsinitiationsfaktor 1A (eIF1A), einem (alpha-)Tubulingen, einem alpha-Actiningen und einem Ecdysonrezeptorgen ausgewählt ist.

10. Verfahren nach einem der Ansprüche 1, 2, 4 bis 9, wobei es sich bei den Genen des pflanzensaftsaugenden Insekts um diejenigen Gene eines pflanzensaftsaugenden Insekts handelt, die durch Verwendung der Primer von einer der SEQ ID NO: 1-4 und SEQ ID NO: 9 und 10 identifiziert werden, oder um Gene von pflanzensaftsaugenden Insekten, die einer der Sequenzen SEQ ID NO: 5 bis 8, SEQ ID NO: 11 oder SEQ ID NO: 12 entsprechen bzw. diese umfassen, sowie die Blattlausgene mit Homologie zu den Sequenzen SEQ ID NO: 5 bis 8, SEQ ID NO: 11 oder SEQ ID NO: 12, wobei zwei Gene homolog sind, wenn sie in einer Sequenz so stark ähnlich sind, dass, wenn mit den beiden Sequenzen ein Alignment erfolgt, die Anzahl Positionen mit identischen Nukleotiden dividiert durch die Anzahl Nukleotide in der kürzeren der Sequenzen mehr als 70% beträgt.

11. Verfahren nach Anspruch 10, wobei mit der dsRNA oder siRNA ein Targeting der A. gossypii-eIFlA-Sequenz von SEQ ID NO: 5 nur in dem Abschnitt von Nukleotidposition 72 bis zum Ende in SEQ ID NO: 5 erfolgt.

12. Verfahren nach einem der Ansprüche 1, 2, 4 bis 9, wobei das Gen des pflanzensaftsaugenden Insekts die Sequenz von einer der SEQ ID NO: 5 bis 8 oder SEQ ID NO: 512 umfasst.

## Revendications

1. Procédé pour rendre silencieux un gène d'un insecte suceur de sève, comprenant l'application, au régime alimentaire ou sur l'alimentation dudit insecte suceur de sève, d'un ARN double brin ou d'un petit ARN interférent sans agents promoteurs de transfection, lequel ARN double brin ou petit ARN interférent étant ciblé vers un gène essentiel d'un insecte suceur de sève.

2. Procédé pour l'identification de la fonction d'un gène, dans un insecte suceur de sève, comprenant l'étape d'application d'un ARN double brin ou d'un petit ARN interférent ciblant un gène de l'insecte suceur de sève vers le régime alimentaire dudit insecte, et d'évaluation des changements phénotypiques ou biochimiques dudit insecte, ledit ARN double brin ou ledit petit ARN interférent étant appliqué sans agents promoteurs de transfection.

3. Procédé pour l'identification de nouvelles cibles pour des composés insecticides, comprenant les étapes de : a) application d'un ARN double brin ou d'un petit ARN interférent, sans agents promoteurs de transfection, sur l'alimentation ou le régime alimentaire d'un insecte suceur de sève ; b) analyse pour déterminer les gènes qui, quand ils sont rendus silencieux, conduisent à une létalité dudit insecte ; c) clonage et caractérisation desdits gènes ainsi analysés ; d) identification des composés qui rompent ou inactivent ledit gène ou l'ARN ou la protéine codée par ce dernier ; et e) mise en contact desdits composés avec ledit insecte, ou ladite alimentation ou ledit régime alimentaire dudit insecte, pour confirmer la nature pesticide dudit composé.

4. Procédé pour obtenir une mortalité significative d'insectes ou une maîtrise significative d'insectes, par le fait que l'on rend silencieux un gène essentiel d'un insecte suceur de sève par application d'un ARN double brin ou d'un petit ARN interférent à l'alimentation dudit insecte, par comparaison avec des insectes témoins alimentés sur un ARN double brin ou un petit ARN interférent ciblant un gène non essentiel ou un gène qui n'est pas exprimé dans l'insecte suceur de sève.

5. Procédé de l'une quelconque des revendications 1, 2 ou 4, dans lequel ledit gène d'insectes suceurs de sève est homologue d'un gène qui, quand il a été partiellement ou complètement rendu silencieux dans un insecte tel que *Drosophila,* conduit à un mutant ayant un phénotype létal, et dans lequel 2 gènes sont homologues quand ils présentent une similitude de séquence dans une mesure telle que, quand les deux séquences sont alignées, le nombre de positions ayant des nucléotides identiques, divisé par le nombre de nucléotides se trouvant dans la plus courte des séquences, est supérieur à 70 %.

6. Procédé de l'une quelconque des revendications 1, 2 ou 4, dans lequel ledit gène d'insectes suceurs de sève est un gène dont le silençage conduit à une diminution de la croissance, du développement, de la reproduction ou de la survie d'un insecte suceur de sève, par comparaison avec des insectes témoins alimentés sur un ARN double brin ou un petit ARN interférent ciblant un gène qui n'est pas exprimé dans l'insecte suceur de sève.

7. Procédé de l'une quelconque des revendications 1, 2, 4 à 6, dans lequel ledit ARN double brin ou petit ARN interférent ne rend pas silencieux les gènes d'une plante, ou d'animaux non cibles, tels que des prédateurs d'insectes suceurs de sève ou des animaux tels que les reptiles, les amphibiens, les oiseaux ou les mammifères .

8. Procédé de l'une quelconque des revendications 1, 2, 4 à 7, dans lequel une partie d'une séquence cible est choisie, qui est présente dans plusieurs insectes suceurs de sève d'une plante hôte, selon une séquence identique ou ayant une identité élevée de séquence, où une séquence ayant une grande identité de séquence se rapporte au fait que le nombre de positions ayant des nucléotides identiques, divisé par le nombre de nucléotides dans la plus courte des séquences, est supérieur à 95 %.

9. Procédé de l'une quelconque des revendications 1, 2, 4 à 8, dans lequel ledit gène d'insectes suceurs de sève est choisi dans le groupe comprenant le facteur d'initiation de la traduction des eucaryotes 1A (elF1A), un gène de l'(alpha)-tubuline, un gène de l'alpha-actinine et un gène du récepteur de l'ecdysone.

10. Procédé de l'une quelconque des revendications 1, 2, 4 à 9, dans lequel lesdits gènes d'insectes suceurs de sève sont les gènes d'insectes suceurs de sève qui sont identifiés par utilisation des amorces ayant l'une quelconque des séquences SEQ ID N° 1-4 et SEQ ID N° 9 et 10, ou les gènes d'insectes suceurs de sève correspondant à l'une quelconque des séquences SEQ ID N° 5 à 8, SEQ ID N° 11 ou SEQ ID N° 12, ou la comprenant, ainsi que les gènes de pucerons, homologues des séquences SEQ ID N° 5 à 8, SEQ ID N° 11 ou SEQ ID N° 12, où 2 gènes sont homologues quand ils présentent une similitude de séquence dans une mesure telle que, quand les deux séquences sont alignées, le nombre de positions ayant des nucléotides identiques, divisé par le nombre de nucléotides dans la plus courte des séquences, est supérieur à 70 %.

11. Procédé de la revendication 10, dans lequel l'ARN double brin ou le petit ARN interférent cible la séquence elF1A de *A*. *gossypii* SEQ ID N° 5, seulement dans la partie allant de la position nucléotidique 72 à l'extrémité de SEQ ID N° 5.

12. Procédé de l'une quelconque des revendications 1, 2, 4 à 9, dans lequel ledit gène d'insecte suceur de sève comprend la séquence de l'une quelconque de SEQ ID N° 5 à 8, ou SEQ ID N° 12.
